# EUROPEAN PATENT APPLICATION

(11) **EP 1 217 074 A1**
(43) Date of publication of application: **26.06.2002**
(21) Application number: 00204693.6
(22) Date of filing: 22.12.2000
(51) Int. Cl.: C12N 15/90, C12N 15/82, C12N 15/81

(54) **Nucleic acid integration in eukaryotes**

(71) Applicant: Universiteit Leiden, 2312 AV Leiden (NL); Stichting Binair Vector Systeem, 2343 RS Oegstgeest (NL)
(72) Inventor: Hooykaas, Paul Jan Jacob, 2343 RS Oegstgeest (NL); Van Attikum, Haico, 2334 EV Leiden (NL); Bundock, Paul, 1106 HJ Amsterdam (NL)
(74) Representative: Prins, Adrianus Willem

(57) **Abstract**

The invention relate to the field of molecular biology and cell biology. It particularly relates to methods to direct integration of a nucleic acid of interest towards homologous recombination and uses thereof. The present invention discloses factors involved in integration of a nucleic acid by illegitimate recombination which provides a method to direct integration of a nucleic acid of interest to a pre-determined site, whereby said nucleic acid has homology at or around the said pre-determined site, in an eukaryote with a preference for non-homologous recombination comprising steering an integration pathway towards homologous recombination. Furthermore, the invention provides a method to direct integration of a nucleic acid of interest to a sub-telomeric and/or telomeric region in an eukaryote with a preference for non-homologous recombination.

SEQUENCE LISTING

<110> Universiteit Leiden
      Stichting Binair Vector Systeem

<120> Nucleic acid integration in eukaryotes

<130> P54997EP00

<140> 00204693.6
<141> 2000-12-22

<160> 34

<170> PatentIn Ver. 2.1

<210> 1
<211> 18
<212> DNA
<213> Artificial Sequence

<220>
<223> Description of Artificial Sequence: primer hdf1p1

<220>
<221> misc_feature
<222> (1)..(18)

<400> 1
gggattgctt taaggtag                                                18


<210> 2
<211> 18
<212> DNA
<213> Artificial Sequence

<220>
<221> misc_feature
<222> (1)..(18)

<220>
<223> Description of Artificial Sequence: primer hdf1p2

<400> 2
caaataccct accctacc                                                18


<210> 3
<211> 21
<212> DNA
<213> Artificial Sequence

<220>
<223> Description of Artificial Sequence: primer dnl4p1

<220>
<221> misc_feature
<222> (1)..(21)

<400> 3
cgtaagattc gccgagtata g                                            21


<210> 4
<211> 21

<212> DNA
<213> Artificial Sequence

<220>
<223> Description of Artificial Sequence: primer dnl4p2

<400> 4
cgtttcaaat gggaccacag c                                                21


<210> 5
<211> 19
<212> DNA
<213> Artificial Sequence

<220>
<223> Description of Artificial Sequence: primer kanmxp1

<220>
<221> misc_feature
<222> (1)..(19)

<400> 5
agactcacgt ttcgaggcc                                                   19


<210> 6
<211> 20
<212> DNA
<213> Artificial Sequence

<220>
<223> Description of Artificial Sequence: primer kanmxp2

<220>
<221> misc_feature
<222> (1)..(20)

<400> 6
tcaccgaggc agttccatag                                                  20


<210> 7
<211> 22
<212> DNA
<213> Artificial Sequence

<220>
<223> Description of Artificial Sequence: primer kanmxp3

<220>
<221> misc_feature
<222> (1)..(22)

<400> 7
tcgcaggtct gcagcgagga gc                                               22


<210> 8
<211> 23
<212> DNA
<213> Artificial Sequence

<220>
<223> Description of Artificial Sequence: primer kanmxp4

<220>
<221> misc_feature
<222> (1)..(23)

<400> 8
tcgcctcgac atcatctgcc cag                                              23


<210> 9
<211> 22
<212> DNA
<213> Artificial Sequence

<220>
<223> Description of Artificial Sequence: primer kanmxp5

<220>
<221> misc_feature
<222> (1)..(22)

<400> 9
tcacatcatg cccctgagct gc                                               22


<210> 10
<211> 31
<212> DNA
<213> Artificial Sequence

<220>
<223> Description of Artificial Sequence: part of a PCR
      fragment derived from a junction sequence

<220>
<221> misc_feature
<222> (1)..(31)
<223> /note="Wherein N stands for any nucleotide sequence"

<400> 10
caggatatat tcaattgtaa atctcncgag g                                     31


<210> 11
<211> 37
<212> DNA
<213> Artificial Sequence

<220>
<223> Description of Artificial Sequence: part of a PCR
      fragment derived from a junction sequence

<220>
<221> misc_feature
<222> (1)..(37)
<223> /note="Wherein N stands for any nucleotide sequence"

<400> 11
attgtattat atattcaatt gtaaatctcn cgaggta                               37


<210> 12
<211> 33
<212> DNA
<213> Artificial Sequence

<220>

<223> Description of Artificial Sequence: part of a PCR
      fragment derived from a junction sequence

<220>
<221> misc_feature
<222> (1)..(33)
<223> /note="Wherein N stands for any nucleotide sequence"

<400> 12
tgtgggtgtg attcaattgt aaatctcncg agg                                    33


<210> 13
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> /note="Wherein N stands for any nucleotide sequence"

<400> 13
gggggcatca gtattcaatt gtaaatctcn cgagg                                  35


<210> 14
<211> 39
<212> DNA
<213> Artificial Sequence

<220>
<223> Description of Artificial Sequence: part of a PCR
      fragment derived from a junction sequence

<220>
<221> misc_feature
<222> (1)..(39)

<400> 14
gaggtagatg tgagagagtg tgtgtgggtg tgaagtcga                              39


<210> 15
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> /note="Wherein N stands for any nucleotide sequence"

<400> 15
tctggtagat atattcaatt gtaaatctcn cgagg                                  35


<210> 16

<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> /note="Wherein N stands for any nucleotide sequence"

<400> 16
cacatatttc tcattcaatt gtaaatctcn cgagg                                35


<210> 17
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> /note="Wherein N stands for any nucleotide sequence"

<400> 17
cgactacttt atatccaatt gtaaatctcn cgagg                                35


<210> 18
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> note="Wherein N stands for any nucleotide sequence"

<400> 18
gaagaaccca ttattcaatt gtaaatctcn cgagg                                35


<210> 19
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> note="Wherein N stands for any nucleotide sequence"

<400> 19
tgggtgtggg ttattcaatt gtaaatctcn cgagg                                35


<210> 20
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> note="Wherein N stands for any nucleotide sequence"

<400> 20
tgggtgtggt gtgttcaatt gtaaatctcn cgagg                                35


<210> 21
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> /note="Wherein N stands for any nucleotide sequence"

<400> 21
tgtgtgggtg tgggtcaatt gtaaatctcn cgagg                                35


<210> 22
<211> 35
<212> DNA
<213> Artificial Sequence

<220>
<223> Description of Artificial Sequence: part of a PCR
      fragment derived from a junction sequence

<220>
<221> misc_feature
<222> (1)..(35)
<223> /note="Wherein N stands for any nucleotide sequence"

<400> 22
cgtcaaggat atattcaatt gtaaatctcn cgagg                                35


<210> 23
<211> 602
<212> PRT
<213> Saccharomyces cerevisiae

<220>
<221> SITE

<222> (1)..(602)
<223> /note="KU 70"

<400> 23
Met Arg Ser Val Thr Asn Ala Phe Gly Asn Ser Gly Glu Leu Asn Asp
1               5                   10                  15

Gln Val Asp Glu Thr Gly Tyr Arg Lys Phe Asp Ile His Glu Gly Ile
            20                  25                  30

Leu Phe Cys Ile Glu Leu Ser Glu Thr Met Phe Lys Glu Ser Ser Asp
        35                  40                  45

Leu Glu Tyr Lys Ser Pro Leu Leu Glu Ile Leu Glu Ser Leu Asp Glu
    50                  55                  60

Leu Met Ser Gln Leu Val Ile Thr Arg Pro Gly Thr Ala Ile Gly Cys
65                  70                  75                  80

Tyr Phe Tyr Tyr Cys Asn Arg Glu Asp Ala Lys Glu Gly Ile Tyr Glu
                85                  90                  95

Leu Phe Pro Leu Arg Asp Ile Asn Ala Thr Phe Met Lys Lys Leu Asn
            100                 105                 110

Asp Leu Leu Glu Asp Leu Ser Ser Gly Arg Ile Ser Leu Tyr Asp Tyr
        115                 120                 125

Phe Met Phe Gln Gln Thr Gly Ser Glu Lys Gln Val Arg Leu Ser Val
    130                 135                 140

Leu Phe Thr Phe Met Leu Asp Thr Phe Leu Glu Glu Ile Pro Gly Gln
145                 150                 155                 160

Lys Gln Leu Ser Asn Lys Arg Val Phe Leu Phe Thr Asp Ile Asp Lys
                165                 170                 175

Pro Gln Glu Ala Gln Asp Ile Asp Glu Arg Ala Arg Leu Arg Arg Leu
            180                 185                 190

Thr Ile Asp Leu Phe Asp Asn Lys Val Asn Phe Ala Thr Phe Phe Ile
        195                 200                 205

Gly Tyr Ala Asp Lys Pro Phe Asp Asn Glu Phe Tyr Ser Asp Ile Leu
    210                 215                 220

Gln Leu Gly Ser His Thr Asn Glu Asn Thr Gly Leu Asp Ser Glu Phe
225                 230                 235                 240

Asp Gly Pro Ser Thr Lys Pro Ile Asp Ala Lys Tyr Ile Lys Ser Arg
                245                 250                 255

Ile Leu Arg Lys Lys Glu Val Lys Arg Ile Met Phe Gln Cys Pro Leu
            260                 265                 270

Ile Leu Asp Glu Lys Thr Asn Phe Ile Val Gly Val Lys Gly Tyr Thr
        275                 280                 285

Met Tyr Thr His Glu Lys Ala Gly Val Arg Tyr Lys Leu Val Tyr Glu
    290                 295                 300

His Glu Asp Ile Arg Gln Glu Ala Tyr Ser Lys Arg Lys Phe Leu Asn
305                 310                 315                 320

Pro Ile Thr Gly Glu Asp Val Thr Gly Lys Thr Val Lys Val Tyr Pro
                325                 330                 335

Tyr Gly Asp Leu Asp Ile Asn Leu Ser Asp Ser Gln Asp Gln Ile Val
            340                 345                 350

Met Glu Ala Tyr Thr Gln Lys Asp Ala Phe Leu Lys Ile Ile Gly Phe
        355                 360                 365

Arg Ser Ser Ser Lys Ser Ile His Tyr Phe Asn Asn Ile Asp Lys Ser
    370                 375                 380

Ser Phe Ile Val Pro Asp Glu Ala Lys Tyr Glu Gly Ser Ile Arg Thr
385                 390                 395                 400

Leu Ala Ser Leu Leu Lys Ile Leu Arg Lys Lys Asp Lys Ile Ala Ile
                405                 410                 415

Leu Trp Gly Lys Leu Lys Ser Asn Ser His Pro Ser Leu Tyr Thr Leu
            420                 425                 430

Ser Pro Ser Ser Val Lys Asp Tyr Asn Glu Gly Phe Tyr Leu Tyr Arg
        435                 440                 445

Val Pro Phe Leu Asp Glu Ile Arg Lys Phe Pro Ser Leu Leu Ser Tyr
    450                 455                 460

Asp Asp Gly Ser Glu His Lys Leu Asp Tyr Asp Asn Met Lys Lys Val
465                 470                 475                 480

Thr Gln Ser Ile Met Gly Tyr Phe Asn Leu Arg Asp Gly Tyr Asn Pro
                485                 490                 495

Ser Asp Phe Lys Asn Pro Leu Leu Gln Lys His Tyr Lys Val Leu His
            500                 505                 510

Asp Tyr Leu Leu Gln Ile Glu Thr Thr Phe Asp Glu Asn Glu Thr Pro
        515                 520                 525

Asn Thr Lys Lys Asp Arg Met Met Arg Glu Asp Asp Ser Leu Arg Lys
    530                 535                 540

Leu Tyr Tyr Ile Arg Asn Lys Ile Leu Glu Ser Glu Lys Ser Glu Asp
545                 550                 555                 560

Pro Ile Ile Gln Arg Leu Asn Lys Tyr Val Lys Ile Trp Asn Met Phe
                565                 570                 575

Tyr Lys Lys Phe Asn Asp Asp Asn Ile Ser Ile Lys Glu Glu Lys Lys
            580                 585                 590

Pro Phe Asp Lys Lys Pro Lys Phe Asn Ile
        595                 600

<210> 24
<211> 609
<212> PRT
<213> Homo sapiens

<220>
<221> SITE
<222> (1)..(609)
<223> /note="KU 70 homologue"

<400> 24
Met Ser Gly Trp Glu Ser Tyr Tyr Lys Thr Glu Gly Asp Glu Glu Ala

1               5                   10                  15

Glu Glu Glu Gln Glu Glu Asn Leu Glu Ala Ser Gly Asp Tyr Lys Tyr
            20                  25                  30

Ser Gly Arg Asp Ser Leu Ile Phe Leu Val Asp Ala Ser Lys Ala Met
        35                  40                  45

Phe Glu Ser Gln Ser Glu Asp Glu Leu Thr Pro Phe Asp Met Ser Ile
    50                  55                  60

Gln Cys Ile Gln Ser Val Tyr Ile Ser Lys Ile Ile Ser Ser Asp Arg
65                  70                  75                  80

Asp Leu Leu Ala Val Val Phe Tyr Gly Thr Glu Lys Asp Lys Asn Ser
                85                  90                  95

Val Asn Phe Lys Asn Ile Tyr Val Leu Gln Glu Leu Asp Asn Pro Gly
            100                 105                 110

Ala Lys Arg Ile Leu Glu Leu Asp Gln Phe Lys Gly Gln Gln Gly Gln
        115                 120                 125

Lys Arg Phe Gln Asp Met Met Gly His Gly Ser Asp Tyr Ser Leu Ser
    130                 135                 140

Glu Val Leu Trp Val Cys Ala Asn Leu Phe Ser Asp Val Gln Phe Lys
145                 150                 155                 160

Met Ser His Lys Arg Ile Met Leu Phe Thr Asn Glu Asp Asn Pro His
                165                 170                 175

Gly Asn Asp Ser Ala Lys Ala Ser Arg Ala Arg Thr Lys Ala Gly Asp
            180                 185                 190

Leu Arg Asp Thr Gly Ile Phe Leu Asp Leu Met His Leu Lys Lys Pro
        195                 200                 205

Gly Gly Phe Asp Ile Ser Leu Phe Tyr Arg Asp Ile Ile Ser Ile Ala
    210                 215                 220

Glu Asp Glu Asp Leu Arg Val His Phe Glu Glu Ser Ser Lys Leu Glu
225                 230                 235                 240

Asp Leu Leu Arg Lys Val Arg Ala Lys Glu Thr Arg Lys Arg Ala Leu
                245                 250                 255

Ser Arg Leu Lys Leu Lys Leu Asn Lys Asp Ile Val Ile Ser Val Gly
            260                 265                 270

Ile Tyr Asn Leu Val Gln Lys Ala Leu Lys Pro Pro Pro Ile Lys Leu
        275                 280                 285

Tyr Arg Glu Thr Asn Glu Pro Val Lys Thr Lys Thr Arg Thr Phe Asn
    290                 295                 300

Thr Ser Thr Gly Gly Leu Leu Leu Pro Ser Asp Thr Lys Arg Ser Gln
305                 310                 315                 320

Ile Tyr Gly Ser Arg Gln Ile Ile Leu Glu Lys Glu Glu Thr Glu Glu
                325                 330                 335

Leu Lys Arg Phe Asp Asp Pro Gly Leu Met Leu Met Gly Phe Lys Pro
            340                 345                 350

Leu Val Leu Leu Lys Lys His His Tyr Leu Arg Pro Ser Leu Phe Val

355                 360                 365

Tyr Pro Glu Glu Ser Leu Val Ile Gly Ser Ser Thr Leu Phe Ser Ala
    370                 375                 380

Leu Leu Ile Lys Cys Leu Glu Lys Glu Val Ala Ala Leu Cys Arg Tyr
385                 390                 395                 400

Thr Pro Arg Arg Asn Ile Pro Pro Tyr Phe Val Ala Leu Val Pro Gln
                405                 410                 415

Glu Glu Glu Leu Asp Asp Gln Lys Ile Gln Val Thr Pro Pro Gly Phe
            420                 425                 430

Gln Leu Val Phe Leu Pro Phe Ala Asp Asp Lys Arg Lys Met Pro Phe
        435                 440                 445

Thr Glu Lys Ile Met Ala Thr Pro Glu Gln Val Gly Lys Met Lys Ala
    450                 455                 460

Ile Val Glu Lys Leu Arg Phe Thr Tyr Arg Ser Asp Ser Phe Glu Asn
465                 470                 475                 480

Pro Val Leu Gln Gln His Phe Arg Asn Leu Glu Ala Leu Ala Leu Asp
                485                 490                 495

Leu Met Glu Pro Glu Gln Ala Val Asp Leu Thr Leu Pro Lys Val Glu
            500                 505                 510

Ala Met Asn Lys Arg Leu Gly Ser Leu Val Asp Glu Phe Lys Glu Leu
        515                 520                 525

Val Tyr Pro Pro Asp Tyr Asn Pro Glu Gly Lys Val Thr Lys Arg Lys
    530                 535                 540

His Asp Asn Glu Gly Ser Gly Ser Lys Arg Pro Lys Val Glu Tyr Ser
545                 550                 555                 560

Glu Glu Glu Leu Lys Thr His Ile Ser Lys Gly Thr Leu Gly Lys Phe
                565                 570                 575

Thr Val Pro Met Leu Lys Glu Ala Cys Arg Ala Tyr Gly Leu Lys Ser
            580                 585                 590

Gly Leu Lys Lys Gln Glu Leu Leu Glu Ala Leu Thr Lys His Phe Gln
        595                 600                 605

Asp

<210> 25
<211> 477
<212> PRT
<213> Arabidopsis thaliana

<220>
<221> SITE
<222> (1)..(477)
<223> /note="KU 70 homologue"

<400> 25
Glu Asn Ser Leu Tyr Ser Ala Leu Trp Val Ala Gln Ala Leu Leu Arg
1               5                   10                  15

Lys Gly Ser Leu Lys Thr Ala Asp Lys Arg Met Phe Leu Phe Thr Asn
            20                  25                  30

Glu Asp Asp Pro Phe Gly Ser Met Arg Ile Ser Val Lys Glu Asp Met
        35                  40                  45

Thr Arg Thr Thr Leu Gln Arg Ala Lys Asp Ala Gln Asp Leu Gly Ile
    50                  55                  60

Ser Ile Glu Leu Leu Pro Leu Ser Gln Pro Asp Lys Gln Phe Asn Ile
65                  70                  75                  80

Thr Leu Phe Tyr Lys Asp Leu Ile Gly Leu Asn Ser Asp Glu Leu Thr
                85                  90                  95

Glu Phe Met Pro Ser Val Gly Gln Lys Leu Glu Asp Met Lys Asp Gln
            100                 105                 110

Leu Lys Lys Arg Val Leu Ala Lys Arg Ile Ala Lys Arg Ile Thr Phe
        115                 120                 125

Val Ile Cys Asp Gly Leu Ser Ile Glu Leu Asn Gly Tyr Ala Leu Leu
    130                 135                 140

Arg Pro Ala Ile Pro Gly Ser Ile Thr Trp Leu Asp Ser Thr Thr Asn
145                 150                 155                 160

Leu Pro Val Lys Val Glu Arg Ser Tyr Ile Cys Thr Asp Thr Gly Ala
                165                 170                 175

Ile Met Gln Asp Pro Ile Gln Arg Ile Gln Pro Tyr Lys Asn Gln Asn
            180                 185                 190

Ile Met Phe Thr Val Glu Glu Leu Ser Gln Val Lys Arg Ile Ser Thr
        195                 200                 205

Gly His Leu Arg Leu Leu Gly Phe Lys Pro Leu Ser Cys Leu Lys Asp
    210                 215                 220

Tyr His Asn Leu Lys Pro Ser Thr Phe Leu Tyr Pro Ser Asp Lys Glu
225                 230                 235                 240

Val Ile Gly Ser Thr Arg Ala Phe Ile Ala Leu His Arg Ser Met Ile
                245                 250                 255

Gln Leu Glu Arg Phe Ala Val Ala Phe Tyr Gly Gly Thr Thr Pro Pro
            260                 265                 270

Arg Leu Val Ala Leu Val Ala Gln Asp Glu Ile Glu Ser Asp Gly Gly
        275                 280                 285

Gln Val Glu Pro Pro Gly Ile Asn Met Ile Tyr Leu Pro Tyr Ala Asn
    290                 295                 300

Asp Ile Arg Asp Ile Asp Glu Leu His Ser Lys Pro Gly Val Ala Xaa
305                 310                 315                 320

Pro Arg Ala Ser Asp Asp Gln Leu Lys Lys Ala Ser Ala Leu Met Arg
                325                 330                 335

Arg Leu Glu Leu Lys Asp Phe Ser Val Cys Gln Phe Ala Asn Pro Ala
            340                 345                 350

Leu Gln Arg His Tyr Ala Ile Leu Gln Ala Ile Ala Leu Asp Glu Asn
        355                 360                 365

Glu Leu Arg Glu Thr Arg Asp Glu Thr Leu Pro Asp Glu Glu Gly Met
    370                 375                 380

Asn Arg Pro Ala Val Val Lys Ala Ile Glu Gln Phe Lys Gln Ser Ile
385                 390                 395                 400

Tyr Gly Asp Asp Pro Asp Glu Glu Ser Asp Ser Gly Ala Lys Glu Lys
                405                 410                 415

Ser Lys Lys Arg Lys Ala Gly Asp Ala Asp Asp Gly Lys Tyr Asp Tyr
            420                 425                 430

Ile Glu Leu Ala Lys Thr Gly Lys Leu Lys Asp Leu Thr Val Val Glu
        435                 440                 445

Leu Lys Thr Tyr Leu Thr Ala Asn Asn Leu Leu Val Ser Gly Lys Lys
    450                 455                 460

Glu Val Leu Ile Asn Arg Ile Leu Thr His Ile Gly Lys
465                 470                 475

<210> 26
<211> 944
<212> PRT
<213> Saccharomyces cerevisiae

<220>
<221> SITE
<222> (1)..(944)
<223> /note="LIG 4"

<400> 26
Met Ile Ser Ala Leu Asp Ser Ile Pro Glu Pro Gln Asn Phe Ala Pro
1               5                   10                  15

Ser Pro Asp Phe Lys Trp Leu Cys Glu Glu Leu Phe Val Lys Ile His
            20                  25                  30

Glu Val Gln Ile Asn Gly Thr Ala Gly Thr Gly Lys Ser Arg Ser Phe
        35                  40                  45

Lys Tyr Tyr Glu Ile Ile Ser Asn Phe Val Glu Met Trp Arg Lys Thr
    50                  55                  60

Val Gly Asn Asn Ile Tyr Pro Ala Leu Val Leu Ala Leu Pro Tyr Arg
65                  70                  75                  80

Asp Arg Arg Ile Tyr Asn Ile Lys Asp Tyr Val Leu Ile Arg Thr Ile
                85                  90                  95

Cys Ser Tyr Leu Lys Leu Pro Lys Asn Ser Ala Thr Glu Gln Arg Leu
            100                 105                 110

Lys Asp Trp Lys Gln Arg Val Gly Lys Gly Gly Asn Leu Ser Ser Leu
        115                 120                 125

Leu Val Glu Glu Ile Ala Lys Arg Arg Ala Glu Pro Ser Ser Lys Ala
    130                 135                 140

Ile Thr Ile Asp Asn Val Asn His Tyr Leu Asp Ser Leu Ser Gly Asp
145                 150                 155                 160

Arg Phe Ala Ser Gly Arg Gly Phe Lys Ser Leu Val Lys Ser Lys Pro
                165                 170                 175

Phe Leu His Cys Val Glu Asn Met Ser Phe Val Glu Leu Lys Tyr Phe
            180                 185                 190

Phe Asp Ile Val Leu Lys Asn Arg Val Ile Gly Gly Gln Glu His Lys
        195                 200                 205

Leu Leu Asn Cys Trp His Pro Asp Ala Gln Asp Tyr Leu Ser Val Ile
    210                 215                 220

Ser Asp Leu Lys Val Val Thr Ser Lys Leu Tyr Asp Pro Lys Val Arg
225                 230                 235                 240

Leu Lys Asp Asp Asp Leu Ser Ile Lys Val Gly Phe Ala Phe Ala Pro
                245                 250                 255

Gln Leu Ala Lys Lys Val Asn Leu Ser Tyr Glu Lys Ile Cys Arg Thr
            260                 265                 270

Leu His Asp Asp Phe Leu Val Glu Glu Lys Met Asp Gly Glu Arg Ile
        275                 280                 285

Gln Val His Tyr Met Asn Tyr Gly Glu Ser Ile Lys Phe Phe Ser Arg
    290                 295                 300

Arg Gly Ile Asp Tyr Thr Tyr Leu Tyr Gly Ala Ser Leu Ser Ser Gly
305                 310                 315                 320

Thr Ile Ser Gln His Leu Arg Phe Thr Asp Ser Val Lys Glu Cys Val
                325                 330                 335

Leu Asp Gly Glu Met Val Thr Phe Asp Ala Lys Arg Arg Val Ile Leu
            340                 345                 350

Pro Phe Gly Leu Val Lys Gly Ser Ala Lys Glu Ala Leu Ser Phe Asn
        355                 360                 365

Ser Ile Asn Asn Val Asp Phe His Pro Leu Tyr Met Val Phe Asp Leu
    370                 375                 380

Leu Tyr Leu Asn Gly Thr Ser Leu Thr Pro Leu Pro Leu His Gln Arg
385                 390                 395                 400

Lys Gln Tyr Leu Asn Ser Ile Leu Ser Pro Leu Lys Asn Ile Val Glu
                405                 410                 415

Ile Val Arg Ser Ser Arg Cys Tyr Gly Val Glu Ser Ile Lys Lys Ser
            420                 425                 430

Leu Glu Val Ala Ile Ser Leu Gly Ser Glu Gly Val Val Leu Lys Tyr
        435                 440                 445

Tyr Asn Ser Ser Tyr Asn Val Ala Ser Arg Asn Asn Asn Trp Ile Lys
    450                 455                 460

Val Lys Pro Glu Tyr Leu Glu Glu Phe Gly Glu Asn Leu Asp Leu Ile
465                 470                 475                 480

Val Ile Gly Arg Asp Ser Gly Lys Lys Asp Ser Phe Met Leu Gly Leu
                485                 490                 495

Leu Val Leu Asp Glu Glu Glu Tyr Lys Lys His Gln Gly Asp Ser Ser
            500                 505                 510

Glu Ile Val Asp His Ser Ser Gln Glu Lys His Ile Gln Asn Ser Arg
        515                 520                 525

Phe Leu His Cys Val Glu Asn Met Ser Phe Val Glu Leu Lys Tyr Phe
180 185 190

Phe Asp Ile Val Leu Lys Asn Arg Val Ile Gly Gly Gln Glu His Lys
195 200 205

Leu Leu Asn Cys Trp His Pro Asp Ala Gln Asp Tyr Leu Ser Val Ile
210 215 220

Ser Asp Leu Lys Val Val Thr Ser Lys Leu Tyr Asp Pro Lys Val Arg
225 230 235 240

Leu Lys Asp Asp Asp Leu Ser Ile Lys Val Gly Phe Ala Phe Ala Pro
245 250 255

Gln Leu Ala Lys Lys Val Asn Leu Ser Tyr Glu Lys Ile Cys Arg Thr
260 265 270

Leu His Asp Asp Phe Leu Val Glu Glu Lys Met Asp Gly Glu Arg Ile
275 280 285

Gln Val His Tyr Met Asn Tyr Gly Glu Ser Ile Lys Phe Phe Ser Arg
290 295 300

Arg Gly Ile Asp Tyr Thr Tyr Leu Tyr Gly Ala Ser Leu Ser Ser Gly
305 310 315 320

Thr Ile Ser Gln His Leu Arg Phe Thr Asp Ser Val Lys Glu Cys Val
325 330 335

Leu Asp Gly Glu Met Val Thr Phe Asp Ala Lys Arg Arg Val Ile Leu
340 345 350

Pro Phe Gly Leu Val Lys Gly Ser Ala Lys Glu Ala Leu Ser Phe Asn
355 360 365

Ser Ile Asn Asn Val Asp Phe His Pro Leu Tyr Met Val Phe Asp Leu
370 375 380

Leu Tyr Leu Asn Gly Thr Ser Leu Thr Pro Leu Pro Leu His Gln Arg
385 390 395 400

Lys Gln Tyr Leu Asn Ser Ile Leu Ser Pro Leu Lys Asn Ile Val Glu
405 410 415

Ile Val Arg Ser Ser Arg Cys Tyr Gly Val Glu Ser Ile Lys Lys Ser
420 425 430

Leu Glu Val Ala Ile Ser Leu Gly Ser Glu Gly Val Val Leu Lys Tyr
435 440 445

Tyr Asn Ser Ser Tyr Asn Val Ala Ser Arg Asn Asn Asn Trp Ile Lys
450 455 460

Val Lys Pro Glu Tyr Leu Glu Glu Phe Gly Glu Asn Leu Asp Leu Ile
465 470 475 480

Val Ile Gly Arg Asp Ser Gly Lys Lys Asp Ser Phe Met Leu Gly Leu
485 490 495

Leu Val Leu Asp Glu Glu Glu Tyr Lys Lys His Gln Gly Asp Ser Ser
500 505 510

Glu Ile Val Asp His Ser Ser Gln Glu Lys His Ile Gln Asn Ser Arg
515 520 525

Arg Arg Val Lys Lys Ile Leu Ser Phe Cys Ser Ile Ala Asn Gly Ile
530 535 540

Ser Gln Glu Glu Phe Lys Glu Ile Asp Arg Lys Thr Arg Gly His Trp
545 550 555 560

Lys Arg Thr Ser Glu Val Ala Pro Pro Ala Ser Ile Leu Glu Phe Gly
565 570 575

Ser Lys Ile Pro Ala Glu Trp Ile Asp Pro Ser Glu Ser Ile Val Leu
580 585 590

Glu Ile Lys Ser Arg Ser Leu Asp Asn Thr Glu Thr Asn Met Gln Lys
595 600 605

Tyr Ala Thr Asn Cys Thr Leu Tyr Gly Gly Tyr Cys Lys Arg Ile Arg
610 615 620

Tyr Asp Lys Glu Trp Thr Asp Cys Tyr Thr Leu Asn Asp Leu Tyr Glu
625 630 635 640

Ser Arg Thr Val Lys Ser Asn Pro Ser Tyr Gln Ala Glu Arg Ser Gln
645 650 655

Leu Gly Leu Ile Arg Lys Lys Arg Lys Arg Val Leu Ile Ser Asp Ser
660 665 670

Phe His Gln Asn Arg Lys Gln Leu Pro Ile Ser Asn Ile Phe Ala Gly
675 680 685

Leu Leu Phe Tyr Val Leu Ser Asp Tyr Val Thr Glu Asp Thr Gly Ile
690 695 700

Arg Ile Thr Arg Ala Glu Leu Glu Lys Thr Ile Val Glu His Gly Gly
705 710 715 720

Lys Leu Ile Tyr Asn Val Ile Leu Lys Arg His Ser Ile Gly Asp Val
725 730 735

Arg Leu Ile Ser Cys Lys Thr Thr Thr Glu Cys Lys Ala Leu Ile Asp
740 745 750

Arg Gly Tyr Asp Ile Leu His Pro Asn Trp Val Leu Asp Cys Ile Ala
755 760 765

Tyr Lys Arg Leu Ile Leu Ile Glu Pro Asn Tyr Cys Phe Asn Val Ser
770 775 780

Gln Lys Met Arg Ala Val Ala Glu Lys Arg Val Asp Cys Leu Gly Asp
785 790 795 800

Ser Phe Glu Asn Asp Ile Ser Glu Thr Lys Leu Ser Ser Leu Tyr Lys
805 810 815

Ser Gln Leu Ser Leu Pro Pro Met Gly Glu Leu Glu Ile Asp Ser Glu
820 825 830

Val Arg Arg Phe Pro Leu Phe Leu Phe Ser Asn Arg Ile Ala Tyr Val
835 840 845

Pro Arg Arg Lys Ile Ser Thr Glu Asp Asp Ile Ile Glu Met Lys Ile
850 855 860

Lys Leu Phe Gly Gly Lys Ile Thr Asp Gln Gln Ser Leu Cys Asn Leu
865 870 875 880

Ile Ile Ile Pro Tyr Thr Asp Pro Ile Leu Arg Lys Asp Cys Met Asn
                885                 890                 895

Glu Val His Glu Lys Ile Lys Glu Gln Ile Lys Ala Ser Asp Thr Ile
            900                 905                 910

Pro Lys Ile Ala Arg Val Val Ala Pro Glu Trp Val Asp His Ser Ile
        915                 920                 925

Asn Glu Asn Cys Gln Val Pro Glu Glu Asp Phe Pro Val Val Asn Tyr
    930                 935                 940

<210> 27
<211> 844
<212> PRT
<213> Homo sapiens

<220>
<221> SITE
<222> (1)..(844)
<223> /note="LIG 4 homologue"

<400> 27

<210> 28
<211> 1219
<212> PRT
<213> Arabidopsis thaliana

<220>
<221> SITE
<222> (1)..(1219)
<223> /note="LIG 4 homologue"

<400> 28

Met Thr Glu Glu Ile Lys Phe Ser Val Leu Val Ser Leu Phe Asn Trp
1               5                   10                  15

Ile Gln Lys Ser Lys Thr Ser Ser Gln Lys Arg Ser Lys Phe Arg Lys
            20                  25                  30

Phe Leu Asp Thr Tyr Cys Lys Pro Ser Asp. Tyr Phe Val Ala Val Arg
        35                  40                  45

Leu Ile Ile Pro Ser Leu Asp Arg Glu Arg Gly Ser Tyr Gly Leu Lys
    50                  55                  60

Glu Ser Val Leu Ala Thr Cys Leu Ile Asp Ala Leu Gly Ile Ser Arg
65                  70                  75                  80

Asp Ala Pro Asp Ala Val Arg Leu Leu Asn Trp Arg Lys Gly Gly Thr
                85                  90                  95

Ala Lys Ala Gly Ala Asn Ala Gly Asn Phe Ser Leu Ile Ala Ala Glu
            100                 105                 110

Val Leu Gln Arg Arg Gln Gly Met Ala Ser Gly Gly Leu Thr Ile Lys
        115                 120                 125

Glu Leu Asn Asp Leu Leu Asp Arg Leu Ala Ser Ser Glu Asn Arg Ala
    130                 135                 140

Glu Lys Thr Leu Val Leu Ser Thr Leu Ile Gln Lys Thr Asn Ala Gln
145                 150                 155                 160

Glu Met Lys Trp Val Ile Arg Ile Ile Leu Lys Asp Leu Lys Leu Gly
                165                 170                 175

Met Ser Glu Lys Ser Ile Phe Gln Glu Phe His Pro Asp Ala Glu Asp
            180                 185                 190

Leu Phe Asn Val Thr Cys Asp Leu Lys Leu Val Cys Glu Lys Leu Arg
        195                 200                 205

Asp Arg His Gln Arg His Lys Arg Gln Asp Ile Glu Val Gly Lys Ala
    210                 215                 220

Val Arg Pro Gln Leu Ala Met Arg Ile Gly Asp Val Asn Ala Ala Trp
225                 230                 235                 240

Lys Lys Leu His Gly Lys Asp Val Val Ala Glu Cys Lys Phe Asp Gly
                245                 250                 255

Asp Arg Ile Gln Ile His Lys Asn Gly Thr Asp Ile His Tyr Phe Ser
            260                 265                 270

Arg Asn Phe Leu Asp His Ser Glu Tyr Ala His Ala Met Ser Asp Leu
        275                 280                 285

Ile Val Gln Asn Ile Leu Val Asp Lys Cys Ile Leu Asp Gly Glu Met
    290                 295                 300

Leu Val Trp Asp Thr Ser Leu Asn Arg Phe Ala Glu Phe Gly Ser Asn
305                 310                 315                 320

Gln Glu Ile Ala Lys Ala Ala Arg Glu Gly Leu Asp Ser His Lys Gln
                325                 330                 335

Leu Cys Tyr Val Ala Phe Asp Val Leu Tyr Val Gly Asp Thr Ser Val
            340                 345                 350

Ile His Gln Ser Leu Lys Glu Arg His Glu Leu Leu Lys Lys Val Val
        355                 360                 365

Lys Pro Leu Lys Gly Arg Leu Glu Val Leu Val Pro Glu Gly Gly Leu
    370                 375                 380

Asn Val His Arg Pro Ser Gly Glu Pro Ser Trp Ser Ile Val Val His
385                 390                 395                 400

Ala Ala Ala Asp Val Glu Arg Phe Phe Lys Glu Thr Val Glu Asn Arg
                405                 410                 415

Asp Glu Gly Ile Val Leu Lys Asp Leu Glu Ser Lys Trp Glu Pro Gly
            420                 425                 430

Asp Arg Ser Gly Lys Trp Met Lys Leu Lys Pro Glu Tyr Ile Arg Ala
        435                 440                 445

Gly Ala Asp Leu Asp Val Leu Ile Ile Gly Gly Tyr Tyr Gly Ser Gly
    450                 455                 460

Arg Arg Gly Gly Glu Val Ala Gln Phe Leu Val Ala Leu Ala Asp Arg
465                 470                 475                 480

Ala Glu Ala Asn Val Tyr Pro Arg Arg Phe Met Ser Phe Cys Arg Val
                485                 490                 495

Gly Thr Gly Leu Ser Asp Asp Glu Leu Asn Thr Val Val Ser Lys Leu
            500                 505                 510

Lys Pro Tyr Phe Arg Lys Asn Glu His Pro Lys Lys Ala Pro Pro Ser
        515                 520                 525

Phe Tyr Gln Val Thr Asn His Ser Lys Glu Arg Pro Asp Val Trp Ile
    530                 535                 540

Asp Ser Pro Glu Lys Ser Ile Ile Leu Ser Ile Thr Ser Asp Ile Arg
545                 550                 555                 560

Thr Ile Arg Ser Glu Val Phe Val Ala Pro Tyr Ser Leu Arg Phe Pro
                565                 570                 575

Arg Ile Asp Lys Val Arg Tyr Asp Lys Pro Trp His Glu Cys Leu Asp
            580                 585                 590

Val Gln Ala Phe Val Glu Leu Val Asn Ser Ser Asn Gly Thr Thr Gln
        595                 600                 605

Lys Gln Lys Glu Ser Glu Ser Thr Gln Asp Asn Pro Lys Val Asn Lys
    610                 615                 620

Ser Ser Lys Arg Gly Glu Lys Lys Asn Val Ser Leu Val Pro Ser Gln
625                 630                 635                 640

Phe Ile Gln Thr Asp Val Ser Asp Ile Lys Gly Lys Thr Ser Ile Phe
                645                 650                 655

Ser Asn Met Ile Phe Tyr Phe Val Asn Val Pro Arg Ser His Ser Leu
            660                 665                 670

Glu Thr Phe His Lys Met Val Val Glu Asn Gly Gly Lys Phe Ser Met
        675                 680                 685

Asn Leu Asn Asn Ser Val Thr His Cys Ile Ala Ala Glu Ser Ser Gly
    690                 695                 700

Ile Lys Tyr Gln Ala Ala Lys Arg Gln Arg Asp Val Ile His Phe Ser
705                 710                 715                 720

Trp Val Leu Asp Cys Cys Ser Arg Asn Lys Met Leu Pro Leu Leu Pro
                725                 730                 735

Lys Tyr Phe Leu His Leu Thr Asp Ala Ser Arg Thr Lys Leu Gln Asp
            740                 745                 750

Asp Ile Asp Glu Phe Ser Asp Ser Tyr Tyr Trp Asp Leu Asp Leu Glu
        755                 760                 765

Gly Leu Lys Gln Val Leu Ser Asn Ala Lys Gln Ser Glu Asp Ser Lys
    770                 775                 780

Ser Ile Asp Tyr Tyr Lys Lys Lys Leu Cys Pro Glu Lys Arg Trp Ser
785                 790                 795                 800

Cys Leu Leu Ser Cys Cys Val Tyr Phe Tyr Pro Tyr Ser Gln Thr Leu
                805                 810                 815

Ser Thr Glu Glu Glu Ala Leu Leu Gly Ile Met Ala Lys Arg Leu Met
            820                 825                 830

Leu Glu Val Leu Met Ala Gly Gly Lys Val Ser Asn Asn Leu Ala His
        835                 840                 845

Ala Ser His Leu Val Val Leu Ala Met Ala Glu Glu Pro Leu Asp Phe
    850                 855                 860

Thr Leu Val Ser Lys Ser Phe Ser Glu Met Glu Lys Arg Leu Leu Leu
865                 870                 875                 880

Lys Lys Arg Leu His Val Val Ser Ser His Trp Leu Glu Glu Ser Leu
                885                 890                 895

Gln Arg Glu Glu Lys Leu Cys Glu Asp Val Tyr Thr Leu Arg Pro Lys
            900                 905                 910

Tyr Met Glu Glu Ser Asp Thr Glu Glu Ser Asp Lys Ser Glu His Asp
        915                 920                 925

Thr Thr Glu Val Ala Ser Gln Gly Ser Ala Gln Thr Lys Glu Pro Ala
    930                 935                 940

Ser Ser Lys Ile Ala Ile Thr Ser Ser Arg Gly Arg Ser Asn Thr Arg
945                 950                 955                 960

Ala Val Lys Arg Gly Arg Ser Ser Thr Asn Ser Leu Gln Arg Val Gln
                965                 970                 975

Arg Arg Arg Gly Lys Gln Pro Ser Lys Ile Ser Gly Asp Glu Thr Glu
            980                 985                 990

Glu Ser Asp Ala Ser Glu Glu Lys Val Ser Thr Arg Leu Ser Asp Ile
        995                 1000                1005

Ala Glu Glu Thr Asp Ser Phe Gly Glu Ala Gln Arg Asn Ser Ser Arg
    1010                1015                1020

Gly Lys Cys Ala Lys Arg Gly Lys Ser Arg Val Gly Gln Thr Gln Arg
1025                1030                1035                1040

Val Gln Arg Ser Arg Arg Gly Lys Lys Ala Ala Lys Ile Gly Gly Asp
                1045                1050                1055

Glu Ser Asp Glu Asn Asp Glu Leu Asp Gly Asn Asn Asn Val Ser Ala
        1060                1065                1070

Asp Ala Glu Glu Gly Asn Ala Ala Gly Arg Ser Val Glu Asn Glu Glu
    1075                1080                1085

Thr Arg Glu Pro Asp Ile Ala Lys Tyr Thr Glu Ser Gln Gln Arg Asp
    1090                1095                1100

Asn Thr Val Ala Val Glu Glu Ala Leu Gln Asp Ser Arg Asn Ala Lys
1105                1110                1115                1120

Thr Glu Met Asp Met Lys Glu Lys Leu Gln Ile His Glu Asp Pro Leu
                1125                1130                1135

Gln Ala Met Leu Met Lys Met Phe Pro Ile Pro Ser Gln Lys Thr Thr
            1140                1145                1150

Glu Thr Ser Asn Arg Thr Thr Gly Glu Tyr Arg Lys Ala Asn Val Ser
        1155                1160                1165

Gly Glu Cys Glu Ser Ser Glu Lys Arg Lys Leu Asp Ala Glu Thr Asp
    1170                1175                1180

Asn Thr Ser Val Asn Ala Gly Ala Glu Ser Asp Val Val Pro Pro Leu
1185                1190                1195                1200

Val Lys Lys Lys Lys Val Ser Tyr Arg Asp Val Ala Gly Glu Leu Leu
                1205                1210                1215

Lys Asp Trp

<210> 29
<211> 692
<212> PRT
<213> Saccharomyces cerevisiae

<220>
<221> SITE
<222> (1)..(692)
<223> /note="MRE 11"

<400> 29
Met Asp Tyr Pro Asp Pro Asp Thr Ile Arg Ile Leu Ile Thr Thr Asp
1               5                   10                  15

Asn His Val Gly Tyr Asn Glu Asn Asp Pro Ile Thr Gly Asp Asp Ser
            20                  25                  30

Trp Lys Thr Phe His Glu Val Met Met Leu Ala Lys Asn Asn Asn Val
        35                  40                  45

Asp Met Val Val Gln Ser Gly Asp Leu Phe His Val Asn Lys Pro Ser
    50                  55                  60

Lys Lys Ser Leu Tyr Gln Val Leu Lys Thr Leu Arg Leu Cys Cys Met
65                  70                  75                  80

Gly Asp Lys Pro Cys Glu Leu Glu Leu Leu Ser Asp Pro Ser Gln Val
                85                  90                  95

Phe His Tyr Asp Glu Phe Thr Asn Val Asn Tyr Glu Asp Pro Asn Phe
            100                 105                 110

Asn Ile Ser Ile Pro Val Phe Gly Ile Ser Gly Asn His Asp Asp Ala
        115                 120                 125

Ser Gly Asp Ser Leu Leu Cys Pro Met Asp Ile Leu His Ala Thr Gly
    130                 135                 140

Leu Ile Asn His Phe Gly Lys Val Ile Glu Ser Asp Lys Ile Lys Val
145                 150                 155                 160

Val Pro Leu Leu Phe Gln Lys Gly Ser Thr Lys Leu Ala Leu Tyr Gly
                165                 170                 175

Leu Ala Ala Val Arg Asp Glu Arg Leu Phe Arg Thr Phe Lys Asp Gly
            180                 185                 190

Gly Val Thr Phe Glu Val Pro Thr Met Arg Glu Gly Glu Trp Phe Asn
        195                 200                 205

Leu Met Cys Val His Gln Asn His Thr Gly His Thr Asn Thr Ala Phe
    210                 215                 220

Leu Pro Glu Gln Phe Leu Pro Asp Phe Leu Asp Met Val Ile Trp Gly
225                 230                 235                 240

His Glu His Glu Cys Ile Pro Asn Leu Val His Asn Pro Ile Lys Asn
                245                 250                 255

Phe Asp Val Leu Gln Pro Gly Ser Ser Val Ala Thr Ser Leu Cys Glu
            260                 265                 270

Ala Glu Ala Gln Pro Lys Tyr Val Phe Ile Leu Asp Ile Lys Tyr Gly
        275                 280                 285

Glu Ala Pro Lys Met Thr Pro Ile Pro Leu Glu Thr Ile Arg Thr Phe
    290                 295                 300

Lys Met Lys Ser Ile Ser Leu Gln Asp Val Pro His Leu Arg Pro His
305                 310                 315                 320

Asp Lys Asp Ala Thr Ser Lys Tyr Leu Ile Glu Gln Val Glu Glu Met
                325                 330                 335

Ile Arg Asp Ala Asn Glu Glu Thr Lys Gln Lys Leu Ala Asp Asp Gly
            340                 345                 350

Glu Gly Asp Met Val Ala Glu Leu Pro Lys Pro Leu Ile Arg Leu Arg
        355                 360                 365

Val Asp Tyr Ser Ala Pro Ser Asn Thr Gln Ser Pro Ile Asp Tyr Gln
    370                 375                 380

Val Glu Asn Pro Arg Arg Phe Ser Asn Arg Phe Val Gly Arg Val Ala
385                 390                 395                 400

Asn Gly Asn Asn Val Val Gln Phe Tyr Lys Lys Arg Ser Pro Val Thr
                405                 410                 415

Arg Ser Lys Lys Ser Gly Ile Asn Gly Thr Ser Ile Ser Asp Arg Asp
            420                 425                 430

Val Glu Lys Leu Phe Ser Glu Ser Gly Gly Glu Leu Glu Val Gln Thr
        435                 440                 445

Leu Val Asn Asp Leu Leu Asn Lys Met Gln Leu Ser Leu Leu Pro Glu
    450                 455                 460

Val Gly Leu Asn Glu Ala Val Lys Lys Phe Val Asp Lys Asp Glu Lys
465                 470                 475                 480

Thr Ala Leu Lys Glu Phe Ile Ser His Glu Ile Ser Asn Glu Val Gly
                485                 490                 495

Ile Leu Ser Thr Asn Glu Glu Phe Leu Arg Thr Asp Asp Ala Glu Glu
            500                 505                 510

Met Lys Ala Leu Ile Lys Gln Val Lys Arg Ala Asn Ser Val Arg Pro
        515                 520                 525

Thr Pro Pro Lys Glu Asn Asp Glu Thr Asn Phe Ala Phe Asn Gly Asn
    530                 535                 540

Gly Leu Asp Ser Phe Arg Ser Ser Asn Arg Glu Val Arg Thr Gly Ser
545                 550                 555                 560

Pro Asp Ile Thr Gln Ser His Val Asp Asn Glu Ser Arg Ile Thr His
                565                 570                 575

Ile Ser Gln Ala Glu Ser Ser Lys Pro Thr Ser Lys Pro Lys Arg Val
            580                 585                 590

Arg Thr Ala Thr Lys Lys Lys Ile Pro Ala Phe Ser Asp Ser Thr Val
        595                 600                 605

Ile Ser Asp Ala Glu Asn Glu Leu Gly Asp Asn Asn Asp Ala Gln Asp
    610                 615                 620

Asp Val Asp Ile Asp Glu Asn Asp Ile Ile Met Val Ser Thr Asp Glu
625                 630                 635                 640

Glu Asp Ala Ser Tyr Gly Leu Leu Asn Gly Arg Lys Thr Lys Thr Lys
                645                 650                 655

Thr Arg Pro Ala Ala Ser Thr Lys Thr Ala Ser Arg Arg Gly Lys Gly
            660                 665                 670

Arg Ala Ser Arg Thr Pro Lys Thr Asp Ile Leu Gly Ser Leu Leu Ala
        675                 680                 685

Lys Lys Arg Lys
    690

<210> 30
<211> 708
<212> PRT
<213> Homo sapiens

<220>
<221> SITE
<222> (1)..(708)
<223> /note="MRE 11 homologue"

<400> 30
   Met Ser Thr Ala Asp Ala Leu Asp Asp Glu Asn Thr Phe Lys Ile Leu
   1               5                   10                  15
   Val Ala Thr Asp Ile His Leu Gly Phe Met Glu Lys Asp Ala Ala Arg
               20                  25                  30
   Gly Asn Asp Thr Phe Val Thr Leu Asp Glu Ile Leu Arg Leu Ala Gln
   35                  40                  45
   Glu Asn Glu Val Asp Phe Ile Leu Leu Gly Gly Asp Leu Phe His Glu
       50                  55                  60
   Asn Lys Pro Ser Arg Lys Thr Leu His Thr Cys Leu Glu Leu Leu Arg
   65                  70                  75                  80
   Lys Tyr Cys Met Gly Asp Arg Pro Val Gln Phe Glu Ile Leu Ser Asp
                   85                  90                  95
   Gln Ser Val Asn Phe Gly Phe Ser Lys Phe Pro Trp Val Asn Tyr Gln
               100                 105                 110
   Asp Gly Asn Leu Asn Ile Ser Ile Pro Val Phe Ser Ile His Gly Asn
           115                 120                 125
   His Asp Asp Pro Thr Gly Ala Asp Ala Leu Cys Ala Leu Asp Ile Leu
       130                 135                 140
   Ser Cys Ala Gly Phe Val Asn His Phe Gly Arg Ser Met Ser Val Glu
   145                 150                 155                 160
   Lys Ile Asp Ile Ser Pro Val Leu Leu Gln Lys Gly Ser Thr Lys Ile
                   165                 170                 175
   Ala Leu Tyr Gly Leu Gly Ser Ile Pro Asp Glu Arg Leu Tyr Arg Met
               180                 185                 190
   Phe Val Asn Lys Lys Val Thr Met Leu Arg Pro Lys Glu Asp Glu Asn
           195                 200                 205
   Ser Trp Phe Asn Leu Phe Val Ile His Gln Asn Arg Ser Lys His Gly
       210                 215                 220
   Ser Thr Asn Phe Ile Pro Glu Gln Phe Leu Asp Asp Phe Ile Asp Leu
   225                 230                 235                 240
   Val Ile Trp Gly His Glu His Glu Cys Lys Ile Ala Pro Thr Lys Asn
                   245                 250                 255
   Glu Gln Gln Leu Phe Tyr Ile Ser Gln Pro Gly Ser Ser Val Val Thr
               260                 265                 270
   Ser Leu Ser Pro Gly Glu Ala Val Lys Lys His Val Gly Leu Leu Arg
           275                 280                 285
   Ile Lys Gly Arg Lys Met Asn Met His Lys Ile Pro Leu His Thr Val
       290                 295                 300
   Arg Gln Phe Phe Met Glu Asp Ile Val Leu Ala Asn His Pro Asp Ile
   305                 310                 315                 320
   Phe Asn Pro Asp Asn Pro Lys Val Thr Gln Ala Ile Gln Ser Phe Cys
                   325                 330                 335
   Leu Glu Lys Ile Glu Glu Met Leu Glu Asn Ala Glu Arg Glu Arg Leu
               340                 345                 350
   Gly Asn Ser His Gln Pro Glu Lys Pro Leu Val Arg Leu Arg Val Asp
           355                 360                 365
   Tyr Ser Gly Gly Phe Glu Pro Phe Ser Val Leu Arg Phe Ser Gln Lys
       370                 375                 380
   Phe Val Asp Arg Val Ala Asn Pro Lys Asp Ile Ile His Phe Phe Arg
   385                 390                 395                 400
   His Arg Glu Gln Lys Glu Lys Thr Gly Glu Glu Ile Asn Phe Gly Lys
                   405                 410                 415
   Leu Ile Thr Lys Pro Ser Glu Gly Thr Thr Leu Arg Val Glu Asp Leu
               420                 425                 430
   Val Lys Gln Tyr Phe Gln Thr Ala Glu Lys Asn Val Gln Leu Ser Leu
           435                 440                 445
   Leu Thr Glu Arg Gly Met Gly Glu Ala Val Gln Glu Phe Val Asp Lys
       450                 455                 460
   Glu Glu Lys Asp Ala Ile Glu Glu Leu Val Lys Tyr Gln Leu Glu Lys
   465                 470                 475                 480
   Thr Gln Arg Phe Leu Lys Glu Arg His Ile Asp Ala Leu Glu Asp Lys
                   485                 490                 495
   Ile Asp Glu Glu Val Arg Arg Phe Arg Glu Thr Arg Gln Lys Asn Thr
               500                 505                 510
   Asn Glu Glu Asp Asp Glu Val Arg Glu Ala Met Thr Arg Ala Arg Ala
           515                 520                 525
   Leu Arg Ser Gln Ser Glu Glu Ser Ala Ser Ala Phe Ser Ala Asp Asp
       530                 535                 540
   Leu Met Ser Ile Asp Leu Ala Glu Gln Met Ala Asn Asp Ser Asp Asp
   545                 550                 555                 560
   Ser Ile Ser Ala Ala Thr Asn Lys Gly Arg Gly Arg Gly Arg Gly Arg
                   565                 570                 575
   Arg Gly Gly Arg Gly Gln Asn Ser Ala Ser Arg Gly Gly Ser Gln Arg
               580                 585                 590
   Gly Arg Ala Phe Lys Ser Thr Arg Gln Gln Pro Ser Arg Asn Val Thr
           595                 600                 605
   Thr Lys Asn Tyr Ser Glu Val Ile Glu Val Asp Glu Ser Asp Val Glu
       610                 615                 620
   Glu Asp Ile Phe Pro Thr Thr Ser Lys Thr Asp Gln Arg Trp Ser Ser
   625                 630                 635                 640
   Thr Ser Ser Ser Lys Ile Met Ser Gln Ser Gln Val Ser Lys Gly Val
                   645                 650                 655
   Asp Phe Glu Ser Ser Glu Asp Asp Asp Asp Asp Pro Phe Met Asn Thr
               660                 665                 670
   Ser Ser Leu Arg Arg Asn Arg Arg Leu Ile Tyr Leu Leu Ala Leu Arg
           675                 680                 685
   Asn Met Gln Asp Thr Gly Lys Met Lys Cys Tyr Lys Leu Arg Val Tyr
       690                 695                 700
   Ser Leu Arg Phe
   705

<210> 31
<211> 720

<212> PRT
<213> Arabidopsis thaliana

<220>
<221> SITE
<222> (1)..(720)
<223> /note="MRE 11 homologue"

<400> 31
Met Ser Arg Glu Asp Phe Ser Asp Thr Leu Arg Val Leu Val Ala Thr
1               5                   10                  15

Asp Cys His Leu Gly Tyr Met Glu Lys Asp Glu Ile Arg Arg His Asp
            20                  25                  30

Ser Phe Lys Ala Phe Glu Glu Ile Cys Ser Ile Ala Glu Glu Lys Gln
        35                  40                  45

Val Asp Phe Leu Leu Leu Gly Gly Asp Leu Phe His Glu Asn Lys Pro
    50                  55                  60

Ser Arg Thr Thr Leu Val Lys Ala Ile Glu Ile Leu Arg Arg His Cys
65                  70                  75                  80

Leu Asn Asp Lys Pro Val Gln Phe Gln Val Val Ser Asp Gln Thr Val
                85                  90                  95

Asn Phe Gln Asn Ala Phe Gly Gln Val Asn Tyr Glu Asp Pro His Phe
            100                 105                 110

Asn Val Gly Leu Pro Val Phe Ser Ile His Gly Asn His Asp Asp Pro
        115                 120                 125

Ala Gly Val Asp Asn Leu Ser Ala Ile Asp Ile Leu Ser Ala Cys Asn
    130                 135                 140

Leu Val Asn Tyr Phe Gly Lys Met Val Leu Gly Gly Ser Gly Val Gly
145                 150                 155                 160

Gln Ile Thr Leu Tyr Pro Ile Leu Met Lys Lys Gly Ser Thr Thr Val
                165                 170                 175

Ala Leu Tyr Gly Leu Gly Asn Ile Arg Asp Glu Arg Leu Asn Arg Met
            180                 185                 190

Phe Gln Thr Pro His Ala Val Gln Trp Met Arg Pro Glu Val Gln Glu
        195                 200                 205

Gly Cys Asp Val Ser Asp Trp Phe Asn Ile Leu Val Leu His Gln Asn
    210                 215                 220

Arg Val Lys Ser Asn Pro Lys Asn Ala Ile Ser Glu His Phe Leu Pro
225                 230                 235                 240

Arg Phe Leu Asp Phe Ile Val Trp Gly His Glu His Glu Cys Leu Ile
                245                 250                 255

Asp Pro Gln Glu Val Ser Gly Met Gly Phe His Ile Thr Gln Pro Gly
            260                 265                 270

Ser Ser Val Ala Thr Ser Leu Ile Asp Gly Glu Ser Lys Pro Lys His
        275                 280                 285

Val Leu Leu Leu Glu Ile Lys Gly Asn Gln Tyr Arg Pro Thr Lys Ile
    290                 295                 300

Pro Leu Thr Ser Val Arg Pro Phe Glu Tyr Thr Glu Ile Val Leu Lys
305                 310                 315                 320

Asp Glu Ser Asp Ile Asp Pro Asn Asp Gln Asn Ser Ile Leu Glu His
                325                 330                 335

Leu Asp Lys Val Val Arg Asn Leu Ile Glu Lys Ala Ser Lys Lys Ala
            340                 345                 350

Val Asn Arg Ser Glu Ile Lys Leu Pro Leu Val Arg Ile Lys Val Asp
        355                 360                 365

Tyr Ser Gly Phe Met Thr Ile Asn Pro Gln Arg Phe Gly Gln Lys Tyr
    370                 375                 380

Val Gly Lys Val Ala Asn Pro Gln Asp Ile Leu Ile Phe Ser Lys Ala
385                 390                 395                 400

Ser Lys Lys Gly Arg Ser Glu Ala Asn Ile Asp Asp Ser Glu Arg Leu
                405                 410                 415

Arg Pro Glu Glu Leu Asn Gln Gln Asn Ile Glu Ala Leu Val Ala Glu
            420                 425                 430

Ser Asn Leu Lys Met Glu Ile Leu Pro Val Asn Asp Leu Asp Val Ala
        435                 440                 445

Leu His Asn Phe Val Asn Lys Asp Asp Lys Leu Ala Phe Tyr Ser Cys
    450                 455                 460

Val Gln Tyr Asn Leu Gln Glu Thr Arg Gly Lys Leu Ala Lys Asp Ser
465                 470                 475                 480

Asp Ala Lys Lys Phe Glu Glu Asp Asp Leu Ile Leu Lys Val Gly Glu
                485                 490                 495

Cys Leu Glu Glu Arg Leu Lys Asp Arg Ser Thr Arg Pro Thr Gly Ser
            500                 505                 510

Ser Gln Phe Leu Ser Thr Gly Leu Thr Ser Glu Asn Leu Thr Lys Gly
        515                 520                 525

Ser Ser Gly Ile Ala Asn Ala Ser Phe Ser Asp Asp Glu Asp Thr Thr
    530                 535                 540

Gln Met Ser Gly Leu Ala Pro Pro Thr Arg Gly Arg Arg Gly Ser Ser
545                 550                 555                 560

Thr Ala Asn Thr Thr Arg Gly Arg Ala Lys Ala Pro Thr Arg Gly Arg
                565                 570                 575

Gly Arg Gly Lys Ala Ser Ser Ala Met Lys Gln Thr Thr Leu Asp Ser
            580                 585                 590

Ser Leu Gly Phe Arg Gln Ser Gln Arg Ser Ala Ser Ala Ala Ala Ser
        595                 600                 605

Ala Ala Phe Lys Ser Ala Ser Thr Ile Gly Glu Asp Asp Val Asp Ser
    610                 615                 620

Pro Ser Ser Glu Glu Val Glu Pro Glu Asp Phe Asn Lys Pro Asp Ser
625                 630                 635                 640

Ser Ser Glu Asp Asp Glu Ser Thr Lys Gly Lys Gly Arg Lys Arg Pro
                645                 650                 655

Ala Thr Thr Lys Arg Gly Arg Gly Arg Gly Ser Gly Thr Ser Lys Arg
            660                 665                 670

Gly Arg Lys Asn Glu Ser Ser Ser Ser Leu Asn Arg Leu Leu Ser Ser
        675                 680                 685

Lys Asp Asp Asp Glu Asp Glu Asp Asp Glu Asp Arg Glu Lys Lys Leu
    690                 695                 700

Asn Lys Ser Gln Pro Arg Val Thr Arg Asn Tyr Gly Ala Leu Arg Arg
705                 710                 715                 720

<210> 32
<211> 1312
<212> PRT
<213> Saccharomyces cerevisiae

<220>
<221> SITE
<222> (1)..(1312)
<223> /note="RAD 50"

<400> 32
Met Ser Ala Ile Tyr Lys Leu Ser Ile Gln Gly Ile Arg Ser Phe Asp
1               5                   10                  15

Ser Asn Asp Arg Glu Thr Ile Glu Phe Gly Lys Pro Leu Thr Leu Ile
            20                  25                  30

Val Gly Met Asn Gly Ser Gly Lys Thr Thr Ile Ile Glu Cys Leu Lys
        35                  40                  45

Tyr Ala Thr Thr Gly Asp Leu Pro Pro Asn Ser Lys Gly Gly Val Phe
    50                  55                  60

Ile His Asp Pro Lys Ile Thr Gly Glu Lys Asp Ile Arg Ala Gln Val
65                  70                  75                  80

Lys Leu Ala Phe Thr Ser Ala Asn Gly Leu Asn Met Ile Val Thr Arg
                85                  90                  95

Asn Ile Gln Leu Leu Met Lys Lys Thr Thr Thr Thr Phe Lys Thr Leu
            100                 105                 110

Glu Gly Gln Leu Val Ala Ile Asn Asn Ser Gly Asp Arg Ser Thr Leu
        115                 120                 125

Ser Thr Arg Ser Leu Glu Leu Asp Ala Gln Val Pro Leu Tyr Leu Gly
    130                 135                 140

Val Pro Lys Ala Ile Leu Glu Tyr Val Ile Phe Cys His Gln Glu Asp
145                 150                 155                 160

Ser Leu Trp Pro Leu Ser Glu Pro Ser Asn Leu Lys Lys Lys Phe Asp
                165                 170                 175

Glu Ile Phe Gln Ala Met Lys Phe Thr Lys Ala Leu Asp Asn Leu Lys
            180                 185                 190

Ser Ile Lys Lys Asp Met Ser Val Asp Ile Lys Leu Leu Lys Gln Ser
        195                 200                 205

Val Glu His Leu Lys Leu Asp Lys Asp Arg Ser Lys Ala Met Lys Leu
    210                 215                 220

Asn Ile His Gln Leu Gln Thr Lys Ile Asp Gln Tyr Asn Glu Glu Val
225                 230                 235                 240

Ser Glu Ile Glu Ser Gln Leu Asn Glu Ile Thr Glu Lys Ser Asp Lys
                245                 250                 255

Leu Phe Lys Ser Asn Gln Asp Phe Gln Lys Ile Leu Ser Lys Val Glu
            260                 265                 270

Asn Leu Lys Asn Thr Lys Leu Ser Ile Ser Asp Gln Val Lys Arg Leu
        275                 280                 285

Ser Asn Ser Ile Asp Ile Leu Asp Leu Ser Lys Pro Asp Leu Gln Asn
    290                 295                 300

Leu Leu Ala Asn Phe Ser Lys Val Leu Met Asp Lys Asn Asn Gln Leu
305                 310                 315                 320

Arg Asp Leu Glu Thr Asp Ile Ser Ser Leu Lys Asp Arg Gln Ser Ser
                325                 330                 335

Leu Gln Ser Leu Ser Asn Ser Leu Ile Arg Arg Gln Gly Glu Leu Glu
            340                 345                 350

Ala Gly Lys Glu Thr Tyr Glu Lys Asn Arg Asn His Leu Ser Ser Leu
        355                 360                 365

Lys Glu Ala Phe Gln His Lys Phe Gln Gly Leu Ser Asn Ile Glu Asn
    370                 375                 380

Ser Asp Met Ala Gln Val Asn His Glu Met Ser Gln Phe Lys Ala Phe
385                 390                 395                 400

Ile Ser Gln Asp Leu Thr Asp Thr Ile Asp Gln Phe Ala Lys Asp Ile
                405                 410                 415

Gln Leu Lys Glu Thr Asn Leu Ser Asp Leu Ile Lys Ser Ile Thr Val
            420                 425                 430

Asp Ser Gln Asn Leu Glu Tyr Asn Lys Lys Asp Arg Ser Lys Leu Ile
        435                 440                 445

His Asp Ser Glu Glu Leu Ala Glu Lys Leu Lys Ser Phe Lys Ser Leu
    450                 455                 460

Ser Thr Gln Asp Ser Leu Asn His Glu Leu Glu Asn Leu Lys Thr Tyr
465                 470                 475                 480

Lys Glu Lys Leu Gln Ser Trp Glu Ser Glu Asn Ile Ile Pro Lys Leu
                485                 490                 495

Asn Gln Lys Ile Glu Glu Lys Asn Asn Glu Met Ile Ile Leu Glu Asn
            500                 505                 510

Gln Ile Glu Lys Phe Gln Asp Arg Ile Met Lys Thr Asn Gln Gln Ala
        515                 520                 525

Asp Leu Tyr Ala Lys Leu Gly Leu Ile Lys Lys Ser Ile Asn Thr Lys
    530                 535                 540

Leu Asp Glu Leu Gln Lys Ile Thr Glu Lys Leu Gln Asn Asp Ser Arg
545                 550                 555                 560

Ile Arg Gln Val Phe Pro Leu Thr Gln Glu Phe Gln Arg Ala Asp Leu
                565                 570                 575

Glu Met Asp Phe Gln Lys Leu Phe Ile Asn Met Gln Lys Asn Ile Ala
            580                 585                 590

Ile Asn Asn Lys Lys Met His Glu Leu Asp Arg Arg Tyr Thr Asn Ala
        595                 600                 605

Leu Tyr Asn Leu Asn Thr Ile Glu Lys Asp Leu Gln Asp Asn Gln Lys
    610                 615                 620

Ser Lys Glu Lys Val Ile Gln Leu Leu Ser Glu Asn Leu Pro Glu Asp
625                 630                 635                 640

Cys Thr Ile Asp Glu Tyr Asn Asp Val Leu Glu Glu Thr Glu Leu Ser
                645                 650                 655

Tyr Lys Thr Ala Leu Glu Asn Leu Lys Met His Gln Thr Thr Leu Glu
            660                 665                 670

Phe Asn Arg Lys Ala Leu Glu Ile Ala Glu Arg Asp Ser Cys Cys Tyr
        675                 680                 685

Leu Cys Ser Arg Lys Phe Glu Asn Glu Ser Phe Lys Ser Lys Leu Leu
    690                 695                 700

Gln Glu Leu Lys Thr Lys Thr Asp Ala Asn Phe Glu Lys Thr Leu Lys
705                 710                 715                 720

Asp Thr Val Gln Asn Glu Lys Glu Tyr Leu His Ser Leu Arg Leu Leu
                725                 730                 735

Glu Lys His Ile Ile Thr Leu Asn Ser Ile Asn Glu Lys Ile Asp Asn
            740                 745                 750

Ser Gln Lys Cys Leu Glu Lys Ala Lys Glu Glu Thr Lys Thr Ser Lys
        755                 760                 765

Ser Lys Leu Asp Glu Leu Glu Val Asp Ser Thr Lys Leu Lys Asp Glu
    770                 775                 780

Lys Glu Leu Ala Glu Ser Glu Ile Arg Pro Leu Ile Glu Lys Phe Thr
785                 790                 795                 800

Tyr Leu Glu Lys Glu Leu Lys Asp Leu Glu Asn Ser Ser Lys Thr Ile
                805                 810                 815

Ser Glu Glu Leu Ser Ile Tyr Asn Thr Ser Glu Asp Gly Ile Gln Thr
            820                 825                 830

Val Asp Glu Leu Arg Asp Gln Gln Arg Lys Met Asn Asp Ser Leu Arg
        835                 840                 845

Glu Leu Arg Lys Thr Ile Ser Asp Leu Gln Met Glu Lys Asp Glu Lys
    850                 855                 860

Val Arg Glu Asn Ser Arg Met Ile Asn Leu Ile Lys Glu Lys Glu Leu
865                 870                 875                 880

Thr Val Ser Glu Ile Glu Ser Ser Leu Thr Gln Lys Gln Asn Ile Asp
                885                 890                 895

Asp Ser Ile Arg Ser Lys Arg Glu Asn Ile Asn Asp Ile Asp Ser Arg
            900                 905                 910

Val Lys Glu Leu Glu Ala Arg Ile Ile Ser Leu Lys Asn Lys Lys Asp
        915                 920                 925

Glu Ala Gln Ser Val Leu Asp Lys Val Lys Asn Glu Arg Asp Ile Gln
        930                 935                 940

Val Arg Asn Lys Gln Lys Thr Val Ala Asp Ile Asn Arg Leu Ile Asp
945                 950                 955                 960

Arg Phe Gln Thr Ile Tyr Asn Glu Val Val Asp Phe Glu Ala Lys Gly
                965                 970                 975

Phe Asp Glu Leu Gln Thr Thr Ile Lys Glu Leu Glu Leu Asn Lys Ala
            980                 985                 990

Gln Met Leu Glu Leu Lys Glu Gln Leu Asp Leu Lys Ser Asn Glu Val
        995                 1000                1005

Asn Glu Glu Lys Arg Lys Leu Ala Asp Ser Asn Asn Glu Glu Lys Asn
    1010                1015                1020

Leu Lys Gln Asn Leu Glu Leu Ile Glu Leu Lys Ser Gln Leu Gln His
1025                1030                1035                1040

Ile Glu Ser Glu Ile Ser Arg Leu Asp Val Gln Asn Ala Glu Ala Glu
                1045                1050                1055

Arg Asp Lys Tyr Gln Glu Glu Ser Leu Arg Leu Arg Thr Arg Phe Glu
            1060                1065                1070

Lys Leu Ser Ser Glu Asn Ala Gly Lys Leu Gly Glu Met Lys Gln Leu
        1075                1080                1085

Gln Asn Gln Ile Asp Ser Leu Thr His Gln Leu Arg Thr Asp Tyr Lys
    1090                1095                1100

Asp Ile Glu Lys Asn Tyr His Lys Glu Trp Val Glu Leu Gln Thr Arg
1105                1110                1115                1120

Ser Phe Val Thr Asp Asp Ile Asp Val Tyr Ser Lys Ala Leu Asp Ser
                1125                1130                1135

Ala Ile Met Lys Tyr His Gly Leu Lys Met Gln Asp Ile Asn Arg Ile
            1140                1145                1150

Ile Asp Glu Leu Trp Lys Arg Thr Tyr Ser Gly Thr Asp Ile Asp Thr
        1155                1160                1165

Ile Lys Ile Arg Ser Asp Glu Val Ser Ser Thr Val Lys Gly Lys Ser
    1170                1175                1180

Tyr Asn Tyr Arg Val Val Met Tyr Lys Gln Asp Val Glu Leu Asp Met
1185                1190                1195                1200

Arg Gly Arg Cys Ser Ala Gly Gln Lys Val Leu Ala Ser Ile Ile Ile
                1205                1210                1215

Arg Leu Ala Leu Ser Glu Thr Phe Gly Ala Asn Cys Gly Val Ile Ala
            1220                1225                1230

Leu Asp Glu Pro Thr Thr Asn Leu Asp Glu Glu Asn Ile Glu Ser Leu
        1235                1240                1245

Ala Lys Ser Leu His Asn Ile Ile Asn Met Arg Arg His Gln Lys Asn
    1250                1255                1260

Phe Gln Leu Ile Val Ile Thr His Asp Glu Lys Phe Leu Gly His Met
1265                1270                1275                1280

Asn Ala Ala Ala Phe Thr Asp His Phe Phe Lys Val Lys Arg Asp Asp
                1285                1290                1295

Arg Gln Lys Ser Gln Ile Glu Trp Val Asp Ile Asn Arg Val Thr Tyr
            1300                1305                1310

<210> 33
<211> 1318
<212> PRT
<213> Homo sapiens

<220>
<221> SITE
<222> (1)..(1318)
<223> /note="RAD 50 homologue"

<400> 33
Met Leu Ile Phe Ser Val Arg Asp Met Phe Ala Lys Met Ser Ile Leu
1               5                   10                  15

Gly Val Arg Ser Phe Gly Ile Glu Asp Lys Asp Lys Gln Ile Ile Thr
            20                  25                  30

Phe Phe Ser Pro Leu Thr Ile Leu Val Gly Pro Asn Gly Ala Gly Lys
        35                  40                  45

Thr Thr Ile Ile Glu Cys Leu Lys Tyr Ile Cys Thr Gly Asp Phe Pro
    50                  55                  60

Pro Gly Thr Lys Gly Asn Thr Phe Val His Asp Pro Lys Val Ala Gln
65                  70                  75                  80

Glu Thr Asp Val Arg Ala Gln Ile Arg Leu Gln Phe Arg Asp Val Asn
                85                  90                  95

Gly Glu Leu Ile Ala Val Gln Arg Ser Met Val Cys Thr Gln Lys Ser
            100                 105                 110

Lys Lys Thr Glu Phe Lys Thr Leu Glu Gly Val Ile Thr Arg Thr Lys
        115                 120                 125

His Gly Glu Lys Val Ser Leu Ser Ser Lys Cys Ala Glu Ile Asp Arg
    130                 135                 140

Glu Met Ile Ser Ser Leu Gly Val Ser Lys Ala Val Leu Asn Asn Val
145                 150                 155                 160

Ile Phe Cys His Gln Glu Asp Ser Asn Trp Pro Leu Ser Glu Gly Lys
                165                 170                 175

Ala Leu Lys Gln Lys Phe Asp Glu Ile Phe Ser Ala Thr Arg Tyr Ile
            180                 185                 190

Lys Ala Leu Glu Thr Leu Arg Gln Val Arg Gln Thr Gln Gly Gln Lys
        195                 200                 205

Val Glu Glu Tyr Gln Met Glu Leu Lys Tyr Leu Lys Gln Tyr Lys Glu
    210                 215                 220

Lys Ala Cys Glu Ile Arg Asp Gln Ile Thr Ser Lys Glu Ala Gln Leu
225                 230                 235                 240

Thr Ser Ser Lys Glu Ile Val Lys Ser Tyr Glu Asn Glu Leu Asp Pro

                245                 250                 255

Leu Lys Asn Arg Leu Lys Glu Ile Glu His Asn Leu Ser Lys Ile Met
            260                 265                 270

Lys Leu Asp Asn Glu Ile Lys Ala Leu Asp Ser Arg Lys Lys Gln Met
        275                 280                 285

Glu Lys Asp Asn Ser Glu Leu Glu Glu Lys Met Glu Lys Val Phe Gln
    290                 295                 300

Gly Thr Asp Glu Gln Leu Asn Asp Leu Tyr His Asn His Gln Arg Thr
305                 310                 315                 320

Val Arg Glu Lys Glu Arg Lys Leu Val Asp Cys His Arg Glu Leu Glu
                325                 330                 335

Lys Leu Asn Lys Glu Ser Arg Leu Leu Asn Gln Glu Lys Ser Glu Leu
            340                 345                 350

Leu Val Glu Gln Gly Arg Leu Gln Leu Gln Ala Asp Arg His Gln Glu
        355                 360                 365

His Ile Arg Ala Arg Asp Ser Leu Ile Gln Ser Leu Ala Thr Gln Leu
    370                 375                 380

Glu Leu Asp Gly Phe Glu Arg Gly Pro Phe Ser Glu Arg Gln Ile Lys
385                 390                 395                 400

Asn Phe His Lys Leu Val Arg Glu Arg Gln Glu Gly Glu Ala Lys Thr
                405                 410                 415

Ala Asn Gln Leu Met Asn Asp Phe Ala Glu Lys Glu Thr Leu Lys Gln
            420                 425                 430

Lys Gln Ile Asp Glu Ile Arg Asp Lys Lys Thr Gly Leu Gly Arg Ile
        435                 440                 445

Ile Glu Leu Lys Ser Glu Ile Leu Ser Lys Lys Gln Asn Glu Leu Lys
    450                 455                 460

Asn Val Lys Tyr Glu Leu Gln Gln Leu Glu Gly Ser Ser Asp Arg Ile
465                 470                 475                 480

Leu Glu Leu Asp Gln Glu Leu Ile Lys Ala Glu Arg Glu Leu Ser Lys
                485                 490                 495

Ala Glu Lys Asn Ser Asn Val Glu Thr Leu Lys Met Glu Val Ile Ser
            500                 505                 510

Leu Gln Asn Glu Lys Ala Asp Leu Asp Arg Thr Leu Arg Lys Leu Asp
        515                 520                 525

Gln Glu Met Glu Gln Leu Asn His His Thr Thr Thr Arg Thr Gln Met
    530                 535                 540

Glu Met Leu Thr Lys Asp Lys Ala Asp Lys Asp Glu Gln Ile Arg Lys
545                 550                 555                 560

Ile Lys Ser Arg His Ser Asp Glu Leu Thr Ser Leu Leu Gly Tyr Phe
                565                 570                 575

Pro Asn Lys Lys Gln Leu Glu Asp Trp Leu His Ser Lys Ser Lys Glu
            580                 585                 590

Ile Asn Gln Thr Arg Asp Arg Leu Ala Lys Leu Asn Lys Glu Leu Ala

595                 600                 605

Ser Ser Glu Gln Asn Lys Asn His Ile Asn Asn Glu Leu Glu Arg Lys
    610                 615                 620

Glu Glu Gln Leu Ser Ser Tyr Glu Asp Lys Leu Phe Asp Val Cys Gly
625                 630                 635                 640

Ser Gln Asp Phe Glu Ser Asp Leu Asp Arg Leu Lys Glu Glu Ile Glu
                645                 650                 655

Lys Ser Ser Lys Gln Arg Ala Met Leu Ala Gly Ala Thr Ala Val Tyr
            660                 665                 670

Ser Gln Phe Ile Thr Gln Leu Thr Asp Glu Asn Gln Ser Cys Cys Pro
        675                 680                 685

Val Cys Gln Arg Val Phe Gln Thr Glu Ala Glu Leu Gln Glu Ala Ile
    690                 695                 700

Ser Asp Leu Gln Ser Lys Leu Arg Leu Ala Pro Asp Lys Leu Lys Ser
705                 710                 715                 720

Thr Glu Ser Glu Leu Lys Lys Lys Glu Lys Arg Arg Asp Glu Met Leu
                725                 730                 735

Gly Leu Ala Pro Met Arg Gln Ser Ile Ile Asp Leu Lys Glu Lys Glu
            740                 745                 750

Ile Pro Glu Leu Arg Asn Lys Leu Gln Asn Val Asn Arg Asp Ile Gln
        755                 760                 765

Arg Leu Lys Asn Asp Ile Glu Glu Gln Glu Thr Leu Leu Gly Thr Ile
    770                 775                 780

Met Pro Glu Glu Glu Ser Ala Lys Val Cys Leu Thr Asp Val Thr Ile
785                 790                 795                 800

Met Glu Arg Phe Gln Met Glu Leu Lys Asp Val Glu Arg Lys Ile Ala
                805                 810                 815

Gln Gln Ala Ala Lys Leu Gln Gly Ile Asp Leu Asp Arg Thr Val Gln
            820                 825                 830

Gln Val Asn Gln Glu Lys Gln Glu Lys Gln His Lys Leu Asp Thr Val
        835                 840                 845

Ser Ser Lys Ile Glu Leu Asn Arg Lys Leu Ile Gln Asp Gln Gln Glu
    850                 855                 860

Gln Ile Gln His Leu Lys Ser Thr Thr Asn Glu Leu Lys Ser Glu Lys
865                 870                 875                 880

Leu Gln Ile Ser Thr Asn Leu Gln Arg Arg Gln Gln Leu Glu Glu Gln
                885                 890                 895

Thr Val Glu Leu Ser Thr Glu Val Gln Ser Leu Tyr Arg Glu Ile Lys
            900                 905                 910

Asp Ala Lys Glu Gln Val Ser Pro Leu Glu Thr Thr Leu Glu Lys Phe
        915                 920                 925

Gln Gln Glu Lys Glu Glu Leu Ile Asn Lys Lys Asn Thr Ser Asn Lys
    930                 935                 940

Ile Ala Gln Asp Lys Leu Asn Asp Ile Lys Glu Lys Val Lys Asn Ile

945                 950                 955                 960

His Gly Tyr Met Lys Asp Ile Glu Asn His Ile Gln Asp Gly Lys Asp
                965                 970                 975

Asp Tyr Met Lys Gln Lys Glu Thr Glu Leu Asn Lys Val Ile Ala Gln
            980                 985                 990

Leu Ser Glu Cys Glu Lys His Lys Glu Lys Ile Asn Glu Asp Met Arg
        995                 1000                1005

Leu Met Arg Gln Asp Ile Asp Thr Gln Lys Ile Gln Glu Arg Trp Leu
    1010                1015                1020

Gln Asp Asn Leu Thr Leu Arg Lys Arg Asn Glu Glu Leu Lys Glu Val
1025                1030                1035                1040

Glu Glu Glu Gly Lys Gln His Leu Lys Glu Met Gly Gln Met Gln Val
                1045                1050                1055

Leu Gln Met Lys Ser Glu His Gln Lys Leu Glu Glu Asn Ile Asp Asn
            1060                1065                1070

Ile Lys Arg Asn His Asn Leu Ala Leu Gly Arg Gln Lys Gly Tyr Glu
        1075                1080                1085

Glu Glu Ile Ile His Phe Lys Lys Glu Leu Arg Glu Pro Gln Phe Arg
    1090                1095                1100

Asp Ala Glu Glu Lys Tyr Arg Glu Met Met Ile Val Met Arg Thr Thr
1105                1110                1115                1120

Glu Leu Val Asn Lys Asp Leu Asp Ile Tyr Tyr Lys Thr Leu Asp Gln
                1125                1130                1135

Ala Ile Met Lys Phe His Ser Met Lys Met Glu Glu Ile Asn Lys Ile
            1140                1145                1150

Ile Arg Asp Leu Trp Arg Ser Thr Tyr Arg Gly Gln Asp Ile Glu Tyr
        1155                1160                1165

Ile Glu Ile Arg Ser Asp Ala Asp Glu Asn Val Ser Ala Ser Asp Lys
    1170                1175                1180

Arg Arg Asn Tyr Asn Tyr Arg Val Val Met Leu Lys Gly Asp Thr Ala
1185                1190                1195                1200

Leu Asp Met Arg Gly Arg Cys Ser Ala Gly Gln Lys Val Leu Ala Ser
                1205                1210                1215

Leu Ile Ile Arg Leu Ala Leu Ala Glu Thr Phe Cys Leu Asn Cys Gly
            1220                1225                1230

Ile Ile Ala Leu Asp Glu Pro Thr Thr Asn Leu Asp Arg Glu Asn Ile
        1235                1240                1245

Glu Ser Leu Ala His Ala Leu Val Glu Ile Ile Lys Ser Arg Ser Gln
    1250                1255                1260

Gln Arg Asn Phe Gln Leu Leu Val Ile Thr His Asp Glu Asp Phe Val
1265                1270                1275                1280

Glu Leu Leu Gly Arg Ser Glu Tyr Val Glu Lys Phe Tyr Arg Ile Lys
                1285                1290                1295

Lys Asn Ile Asp Gln Cys Ser Glu Ile Val Lys Cys Ser Val Ser Ser

1300                1305                1310

Leu Gly Phe Asn Val His
        1315

<210> 34
<211> 1292
<212> PRT
<213> Arabidopsis thaliana

<220>
<221> SITE
<222> (1)..(1292)
<223> /note="RAD 50 homologue"

<400> 34
Met Ser Thr Val Asp Lys Met Leu Ile Lys Gly Ile Arg Ser Phe Asp
1               5                   10                  15

Pro Glu Asn Lys Asn Val Val Thr Phe Phe Arg Pro Leu Thr Leu Ile
            20                  25                  30

Val Gly Ala Asn Gly Ala Gly Lys Thr Thr Ile Ile Glu Cys Leu Lys
        35                  40                  45

Val Ser Cys Thr Gly Glu Leu Pro Pro Asn Ala Arg Ser Gly His Ser
    50                  55                  60

Phe Ile His Asp Pro Lys Val Ala Gly Glu Thr Glu Thr Lys Ala Gln
65                  70                  75                  80

Ile Lys Leu Arg Phe Lys Thr Ala Ala Gly Lys Asp Val Val Cys Ile
                85                  90                  95

Arg Ser Phe Gln Leu Thr Gln Lys Ala Ser Lys Met Glu Tyr Lys Ala
            100                 105                 110

Ile Glu Ser Val Leu Gln Thr Ile Asn Pro His Thr Gly Glu Lys Val
        115                 120                 125

Cys Leu Ser Tyr Arg Cys Ala Asp Met Asp Arg Glu Ile Pro Ala Leu
    130                 135                 140

Met Gly Val Ser Lys Ala Ile Leu Glu Asn Val Ile Phe Val His Gln
145                 150                 155                 160

Asp Glu Ser Asn Trp Pro Leu Gln Asp Pro Ser Thr Leu Lys Lys Lys
                165                 170                 175

Phe Asp Asp Ile Phe Ser Ala Thr Arg Tyr Thr Lys Ala Leu Glu Val
            180                 185                 190

Ile Lys Lys Leu His Lys Asp Gln Ala Gln Glu Ile Lys Thr Phe Lys
        195                 200                 205

Leu Lys Leu Glu Asn Leu Gln Thr Leu Lys Asp Ala Ala Tyr Lys Leu
    210                 215                 220

Arg Glu Ser Ile Ala Gln Asp Gln Glu Arg Thr Glu Ser Ser Lys Val
225                 230                 235                 240

Gln Met Leu Glu Leu Glu Thr Ser Val Gln Lys Val Asp Ala Glu Val
                245                 250                 255

His Asn Lys Glu Met Met Leu Lys Asp Leu Arg Lys Leu Gln Asp Gln
            260                 265                 270

Val Ser Ile Lys Thr Ala Glu Arg Ser Thr Leu Phe Lys Glu Gln Gln
        275                 280                 285

Arg Gln Tyr Ala Ala Leu Pro Glu Glu Asn Glu Asp Thr Ile Glu Glu
    290                 295                 300

Leu Lys Glu Trp Lys Ser Lys Phe Glu Glu Arg Leu Ala Leu Leu Gly
305                 310                 315                 320

Thr Lys Ile Arg Lys Met Glu Arg Glu Met Val Asp Thr Glu Thr Thr
                325                 330                 335

Ile Ser Ser Leu His Asn Ala Lys Thr Asn Tyr Met Leu Glu Ile Ser
            340                 345                 350

Lys Leu Gln Thr Glu Ala Glu Ala His Met Leu Leu Lys Asn Glu Arg
        355                 360                 365

Asp Ser Thr Ile Gln Asn Ile Phe Phe His Tyr Asn Leu Gly Asn Val
    370                 375                 380

Pro Ser Thr Pro Phe Ser Thr Glu Val Val Leu Asn Leu Thr Asn Arg
385                 390                 395                 400

Ile Lys Ser Arg Leu Gly Glu Leu Glu Met Asp Leu Leu Asp Lys Lys
                405                 410                 415

Lys Ser Asn Glu Thr Ala Leu Ser Thr Ala Trp Asp Cys Tyr Met Asp
            420                 425                 430

Ala Asn Asp Arg Trp Lys Ser Ile Glu Ala Gln Lys Arg Ala Lys Asp
        435                 440                 445

Glu Ile Lys Met Gly Ile Ser Lys Arg Ile Glu Glu Lys Glu Ile Glu
    450                 455                 460

Arg Asp Ser Phe Glu Phe Glu Ile Ser Thr Val Asp Val Lys Gln Thr
465                 470                 475                 480

Asp Glu Arg Glu Lys Gln Val Gln Val Glu Leu Glu Arg Lys Thr Lys
                485                 490                 495

Gln Asn Ser Glu Arg Gly Phe Glu Ser Lys Ile Glu Gln Lys Gln His
            500                 505                 510

Glu Ile Tyr Ser Leu Glu His Lys Ile Lys Thr Leu Asn Arg Glu Arg
        515                 520                 525

Asp Val Met Ala Gly Asp Ala Glu Asp Arg Leu Leu Thr Arg Ile Asp
    530                 535                 540

Glu Cys Lys Asp Arg Ile Arg Gly Val Leu Lys Gly Arg Leu Pro Pro
545                 550                 555                 560

Glu Lys Asp Met Lys Arg Glu Ile Val Gln Ala Leu Arg Ser Ile Glu
                565                 570                 575

Arg Glu Tyr Asp Asp Leu Ser Leu Lys Ser Arg Glu Ala Glu Lys Glu
            580                 585                 590

Val Asn Met Leu Gln Met Lys Ile Gln Glu Val Asn Asn Ser Leu Phe
        595                 600                 605

Lys His Asn Lys Asp Thr Glu Ser Arg Lys Arg Tyr Ile Glu Ser Lys
    610                 615                 620

Leu Gln Ala Leu Lys Gln Glu Ser Val Thr Ile Asp Ala Tyr Pro Lys
625                 630                 635                 640

Leu Leu Glu Ser Ala Lys Asp Lys Arg Asp Asp Arg Lys Arg Glu Tyr
                645                 650                 655

Asn Met Ala Asn Gly Met Arg Gln Met Phe Glu Pro Phe Glu Lys Arg
            660                 665                 670

Ala Arg Gln Glu His Ser Cys Pro Cys Cys Glu Arg Ser Phe Thr Ala
        675                 680                 685

Asp Glu Glu Ala Ser Phe Ile Lys Lys Gln Arg Val Lys Ala Ser Ser
    690                 695                 700

Thr Gly Glu His Leu Lys Ala Leu Ala Val Glu Ser Ser Asn Ala Asp
705                 710                 715                 720

Ser Val Phe Gln Gln Leu Asp Lys Leu Arg Ala Val Phe Glu Glu Tyr
                725                 730                 735

Ser Lys Leu Thr Thr Glu Ile Ile Pro Leu Ala Glu Lys Thr Leu Gln
            740                 745                 750

Glu His Thr Glu Glu Leu Gly Gln Lys Ser Glu Ala Leu Asp Asp Val
        755                 760                 765

Leu Gly Ile Ser Ala Gln Ile Lys Ala Asp Lys Asp Ser Ile Glu Ala
    770                 775                 780

Leu Val Gln Pro Leu Glu Asn Ala Asp Arg Ile Phe Gln Glu Ile Val
785                 790                 795                 800

Ser Tyr Gln Lys Gln Ile Glu Asp Leu Glu Tyr Lys Leu Asp Phe Arg
                805                 810                 815

Gly Leu Gly Val Lys Thr Met Glu Glu Ile Gln Ser Glu Leu Ser Ser
            820                 825                 830

Leu Gln Ser Ser Lys Asp Lys Leu His Gly Glu Leu Glu Lys Leu Arg
        835                 840                 845

Asp Asp Gln Ile Tyr Met Glu Arg Asp Ile Ser Cys Leu Gln Ala Arg
    850                 855                 860

Trp His Ala Val Arg Glu Glu Lys Ala Lys Ala Ala Asn Leu Leu Arg
865                 870                 875                 880

Asp Val Thr Lys Ala Glu Glu Asp Leu Glu Arg Leu Ala Glu Glu Lys
                885                 890                 895

Ser Gln Leu Asp Leu Asp Val Lys Tyr Leu Thr Glu Ala Leu Gly Pro
            900                 905                 910

Leu Ser Lys Glu Lys Glu Gln Leu Leu Ser Asp Tyr Asn Asp Met Lys
        915                 920                 925

Ile Arg Arg Asn Gln Glu Tyr Glu Glu Leu Ala Glu Lys Lys Arg Asn
    930                 935                 940

Tyr Gln Gln Glu Val Glu Ala Leu Leu Lys Ala Ser Tyr Lys Ile Asn
945                 950                 955                 960

Asp Cys Phe Thr Arg Tyr His Asp Leu Lys Lys Gly Glu Arg Leu Asp
                965                 970                 975

Asp Ile Gln Glu Lys Gln Arg Leu Ser Asp Ser Gln Leu Gln Ser Cys
            980                 985                 990

Glu Ala Arg Lys Asn Glu Leu Ala Gly Glu Leu Asn Arg Asn Lys Asp
        995                 1000                1005

Leu Met Arg Asn Gln Asp Gln Leu Arg Arg Asn Ile Glu Asp Asn Leu
    1010                1015                1020

Asn Tyr Arg Thr Thr Lys Ala Lys Val Glu Glu Leu Thr Arg Glu Ile
1025                1030                1035                1040

Glu Ser Leu Glu Glu Gln Ile Leu Asn Ile Gly Gly Ile Ala Ala Val
                1045                1050                1055

Glu Ala Glu Ile Val Lys Ile Leu Arg Glu Arg Glu Arg Leu Leu Ser
            1060                1065                1070

Glu Leu Asn Arg Cys Arg Gly Thr Val Ser Val Tyr Glu Ser Ser Ile
        1075                1080                1085

Ser Lys Asn Arg Val Glu Leu Lys Gln Ala Gln Tyr Lys Asp Ile Asp
    1090                1095                1100

Lys Arg His Phe Asp Gln Leu Ile Gln Leu Lys Thr Thr Glu Met Ala
1105                1110                1115                1120

Asn Lys Asp Leu Asp Arg Tyr Tyr Asn Ala Leu Asp Lys Ala Leu Met
                1125                1130                1135

Arg Phe His Thr Met Lys Met Glu Glu Ile Asn Lys Ile Ile Arg Glu
            1140                1145                1150

Leu Trp Gln Gln Thr Tyr Arg Gly Gln Asp Met Asp Tyr Ile Arg Ile
        1155                1160                1165

His Ser Asp Ser Glu Gly Ala Gly Thr Arg Ser Tyr Ser Tyr Lys Val
    1170                1175                1180

Leu Met Gln Thr Gly Asp Thr Glu Leu Glu Met Arg Gly Arg Cys Ser
1185                1190                1195                1200

Ala Gly Gln Lys Val Leu Ala Ser Leu Ile Ile Arg Leu Ala Leu Ala
                1205                1210                1215

Glu Thr Phe Cys Leu Asn Cys Gly Ile Leu Ala Leu Asp Glu Pro Thr
            1220                1225                1230

Thr Asn Leu Asp Gly Pro Asn Ser Glu Ser Leu Ala Gly Ala Leu Leu
        1235                1240                1245

Arg Ile Met Glu Asp Arg Lys Gly Gln Glu Asn Phe Gln Leu Ile Val
    1250                1255                1260

Ile Thr His Asp Glu Arg Phe Ala Gln Met Ile Gly Gln Arg Gln His
1265                1270                1275                1280

Ala Glu Lys Tyr Tyr Arg Val Ala Lys Asp Asp Met
                1285                1290

## Description

The invention relates to the field of molecular biology and cell biology. It particularly relates to methods to direct integration towards homologous recombination and uses thereof. Several methods are known to transfer nucleic acids to, in particular, eukaryotic cells. In some methods the nucleic acid of interest is transferred to the cytoplasm of the cell, in some the nucleic acid of interest is integrated into the genome of the host. Many different vehicles for transfer of the nucleic acid are known. For different kinds of cells, different systems can be used, although many systems are more widely applicable than just a certain kind of cells. In plants, e.g., a system based on *Agrobacterium tumefaciens* is often applied. This system is one of the systems that can be used according to the invention.

The soil bacterium *Agrobacterium tumefaciens* is able to transfer part of its tumor-inducing (Ti) plasmid, the transferred (T-) DNA, to plant cells. This results in crown gall tumor formation on plants due to expression of onc-genes, which are present on the T-DNA. Virulence (*vir*) genes, located elsewhere on the Ti-plasmid, mediate T-DNA transfer to the plant cell. Some Vir proteins accompany the T-DNA during its transfer to the plant cell to protect the T-DNA and to mediate its transfer to the plant nucleus. Once in the plant nucleus, the T-DNA is integrated at a random position into the plant genome (reviewed by Hooykaas and Beijersbergen 1994, Hansen and Chilton 1999). Removal of the *onc*-genes from the T-DNA does not inactivate T-DNA transfer. T-DNA, disarmed in this way, is now the preferred vector for the genetic modification of plants.

Although much is known about the transformation process, not much is known about the process by which the T-DNA is integrated into the plant genome. It is likely that plant enzymes mediate this step of the transformation process (Bundock et al. 1995). The integration pattern of T-DNA in transformed plants has been extensively studied (Matsumoto et al. 1990, Gheysen et al. 1991, Mayerhofer et al. 1991). The results indicated that T-DNA integrates via illegitimate recombination (IR) (also called non-homologous recombination, both terms may be used interchangeable herein), a process which can join two DNA molecules that share little or no homology (here the T-DNA and plant target DNA). Even T-DNA molecules in which a large segment of homologous plant DNA was present, integrated mainly by IR and only with very low frequency (1:10⁴-10⁵) by homologous recombination (HR) (Offringa et al. 1990).

Recently, it was shown that *Agrobacterium* is not only able to transfer its T-DNA to plant cells, but also to other eukaryotes, including the yeast *S,cerevisiae* (Bundock et al. 1995) and a wide variety of filamentous fungi (deGroot et al. 1998). In *S.cerevisiae*, T-DNA carrying homology with the yeast genome integrates via HR (Bundock et al. 1995). However, T-DNA lacking any homology with the *S.cerevisiae* genome becomes integrated at random positions in the genome by the same IR process as is used in plants (Bundock and Hooykaas 1996). Apparently, eukaryotic cells have at least two separate pathways (one via homologous and one via non-homologous recombination) through which nucleic acids (in particular of course DNA), can be integrated into the host genome. The site of integration into a host cell genome is important with respect to the likelihood of transcription and/or expression of the integrated nucleic acid. The present invention provides methods and means to direct nucleic acid integration to a predetermined site through steering integration towards the homologous recombination pathway. The present invention arrives at such steering either by enhancing the HR pathway, or by inhibiting (meaning reducing) the IR pathway.

Host factors involved in the integration of nucleic acid by IR have so far not been identified. The present invention discloses such factors which enables the design of methods for their (temporary) inhibition, so that integration of nucleic acid by IR is prevented, shifting the integration process towards HR and facilitating the isolation of a host cell with nucleic acid integrated by HR at a predetermined site. This is extremely important, since there is no method available yet for easy and precise genetic modification of a host cell using HR (gene targeting). Of course the actual site of integration is then determined by homology of the nucleic acid of interest with said site.

In a first embodiment the invention provides a method to direct nucleic acid integration of a nucleic acid of interest to a pre-determined site, whereby said nucleic acid has homology at or around the said pre-determined site, in an eukaryote with a preference for non-homologous recombination comprising steering an integration pathway towards homologous recombination. Integration is a complex process wherein a nucleic acid sequence becomes part of the genetic material of a host cell. One step in the process of nucleic acid integration is recombination; via recombination nucleic acid sequences are exchanged and the introduced nucleic acid becomes part of the genetic material of a host cell. In principle two different ways of recombination are possible: homologous and illegitimate or non-homologous recombination. Most (higher) eukaryotes do not or at least not significantly practise homologous recombination although the essential proteins to accomplish such a process are available. One reason for this phenomenon is that frequent use of homologous recombination in (higher) eukaryotes could lead to undesirable chromosomal rearrangements due to the presence of repetitive nucleic acid sequences. To accomplish homologous recombination via a method according to the invention, it is important to provide a nucleic acid which has homology with a pre-determined site. It is clear to a person skilled in the art that the percentage of homology and the length of (a) homologous region(s) play an important role in the process of homologous recombination. The percentage of homology is preferably close to 100%. A person skilled in the art is aware of the fact that lower percentage of homology are also used in the field of homologous recombination, but dependent on, for example, the regions of homology and their overall distribution, lead to a lower efficiency of homologous recombination but are still useful and therefore included in the present invention. Furthermore, the length of a (nearly) homologous region is approximately 3 kb which is sufficient to direct homologous recombination. At least one homologous region is necessary for recombination but more preferably 2 homologous regions flanking the nucleic acid of interest are used for targeted integration. The researcher skilled in the art knows how to select the proper percentage of homology, the length of homology and the amount of homologous regions. By providing such a homology a nucleic acid is integrated at every desired position within the genetic material of a host cell. It is clear to a person skilled in the art that the invention as disclosed herein is used to direct any nucleic acid (preferably DNA) to any pre-determined site as long as the length of homology and percentage of homology are high enough to provide homologous recombination. A pre-determined site is herein defined as a site within the genetic material contained by a host cell to which a nucleic acid with homology to this same site is integrated with a method according to the invention. It was not until the present invention that a nucleic acid is integrated at every desired position and therefore a method according to the invention is applied, for example, to affect the gene function in various ways, not only for complete inactivation but also to mediate changes in the expression level or in the regulation of expression, changes in protein activity or the subcellular targeting of an encoded protein. Complete inactivation, which can usually not be accomplished by existing methods such as antisense technology or RNAi technology (Zrenner et al, 1993), is useful for instance for the inactivation of genes controlling undesired side branches of metabolic pathways, for instance to increase the quality of bulk products such as starch, or to increase the production of specific secondary metabolites or to inhibit formation of unwanted metabolites. Also to inactivate genes controlling senescence in fruits and flowers or that determine flower pigments. Replacement of existing regulatory sequences by alternative regulatory sequences is used to alter expression of in situ modified genes to meet requirements, (e.g. expression in response to particular physical conditions such as light, drought or pathogen infection, or in response to chemical inducers), or depending on the developmental state (e.g. in a storage organ, or in fruits or seeds) or on tissue or cell types. Also a method according to the invention is used to effectuate predictable expression of transgenes encoding novel products, for example by replacing existing coding sequences of genes giving a desired expression profile by those for a desired novel product. For example to produce proteins of medicinal or industrial value in the seeds of plants the coding sequence of a strongly expressed storage protein may be replaced by that of the desired protein. As another example existing coding sequences are modified so that the protein encoded has optimized characteristics for instance to make a plant herbicide tolerant, to produce storage proteins with enhanced nutritional value, or to target a protein of interest to an organelle or to secrete it to the extracellular space. As yet another example eukaryotic cells (including yeast, fungi, plant and mammalian cells) are provided with a gene encoding a protein of interest integrated into the genome at a site which ensures high expression levels. As another example the nucleic acid of interest can be part of a gene delivery vehicle to deliver a gene of interest to a eukaryotic cell *in vitro* or *in vivo*. In this way a defect p53 can be replaced by an intact p53. In this way a tumoricidal gene can be delivered to a pre-determined site present only in e.g. proliferating cells, or present only in tumor cells, e.g. to the site where a tumor antigen is expressed from. Gene delivery vehicles are well known in the art and include adenoviral vehicles, retroviral vehicles, non-viral vehicles such as liposomes, etc. As another example the invention is used to produce transgenic organisms. Knock-out transgenics are already produced by homologous recombination methods. The present invention improves the efficiency of such methods. Also transgenics with desired properties are made.

In another embodiment the invention provides a method to direct nucleic acid integration to a pre-determined site, whereby said nucleic acid has homology at or around the said pre-determined site, in an eukaryote with a preference for non-homologous recombination comprising steering an integration pathway towards homologous recombination by providing a mutant of a component involved in non-homologous recombination. Methods to identify components involved in non-homologous recombination are outlined in the present description wherein *S.cerevisiae* was used as a model system. To this end several yeast derivatives defective for genes known to be involved in various recombination processes were constructed and the effect of the mutations on T-DNA integration by either HR or IR was tested. The results as disclosed herein show that the proteins encoded by *YKU70*, *RAD50*, *MRE11*, *XRS2*, *LIG4* and *SIR4* play an essential role in DNA integration by IR but not by HR. It is clear to a person skilled in the art that different mutants of a component involved in non-homologous recombination exist. Examples are deletion mutants, knock-out (for example via insertion) mutants or point mutants. Irrespective of the kind of mutant it is important that a component involved in non-homologous recombination is no longer capable or at least significantly less capable to perform it's function in the process of non-homologous recombination. As disclosed herein disruption of *YKU70*, *RAD50, MRE11*, *XRS2*, *LIG4* and *SIR4* did not affect the frequency of DNA integration by HR, showing that these genes are not involved in DNA integration by HR, but only in DNA integration by IR. In another embodiment the invention provides a method to direct integration of a nucleic acid of interest to a subtelomeric and/or telomeric region in an eukaryote with a preference for non-homologous recombination by providing a mutant of a component involved in non-homologous recombination. A telomeric region is defined herein as a region containing repetitive sequences which is located at the end of a chromosome. Sub-telomeric region is herein defined as a region flanking the telomeric region. As an example is disclosed herein that in yeast strains carrying disruptions of *RAD50*, *MRE11 or XRS2* the distribution of integrated DNA copies is altered when compared to wildtype. DNA becomes preferentially integrated in telomeres or subtelomeric regions in the *rad50*, *mre11* and *xrs2* mutants. A great advantage of integration of DNA copies in telomeres or subtelomeric regions instead of integration elsewhere in the genomic material is that there is no danger for host genes being mutated or inactivated by a DNA insertion. When in plants deficient for *RAD50*, *MRE11* or *XRS2* DNA copies also integrate into telomeres or telomeric regions, such plants are used for telomeric targeting of T-DNA in transformation experiments to prevent additional insertion mutations from random T-DNA integration into the plant genome.

In yet another embodiment the invention provides a method to direct nucleic acid integration to a pre-determined site, whereby said nucleic acid has homology at or around the said pre-determined site, in an eukaryote with a preference for non-homologous recombination comprising steering an integration pathway towards homologous recombination by partially or more preferably completely inhibiting a component involved in non-homologous recombination. Partial or complete inhibition of a component involved in non-homologous recombination is obtained by different methods, for example by an antibody directed against such a component or a chemical inhibitor or a protein inhibitor or peptide inhibitor or an antisense molecule or an RNAi molecule. Irrespective of the kind of (partial or more preferably complete) inhibition it is important that a component involved in non-homologous recombination is no longer capable or at least significantly less capable to perform it's function in the process of non-homologous recombination. In yet another embodiment the invention provides a method to direct integration of a nucleic acid of interest to a sub-telomeric and/or telomeric region in an eukaryote with a preference for non-homologous recombination by partially or more preferably completely inhibiting a component involved in non-homologous recombination.

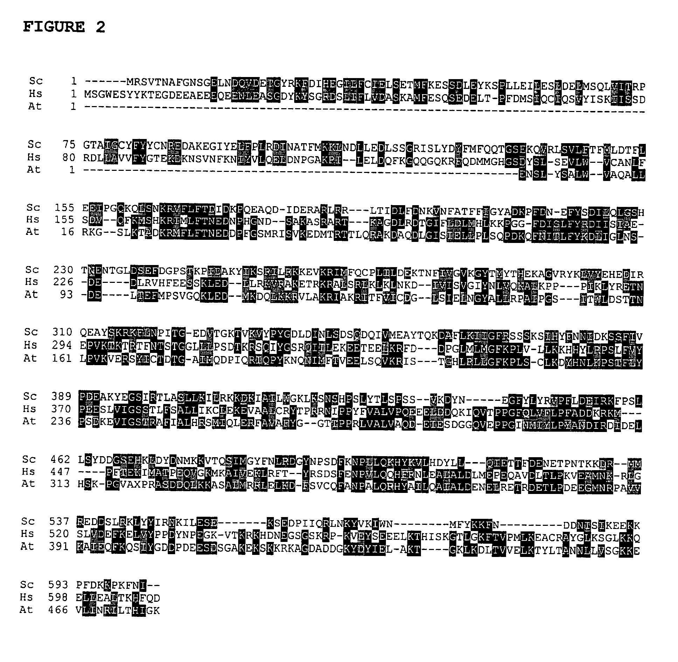

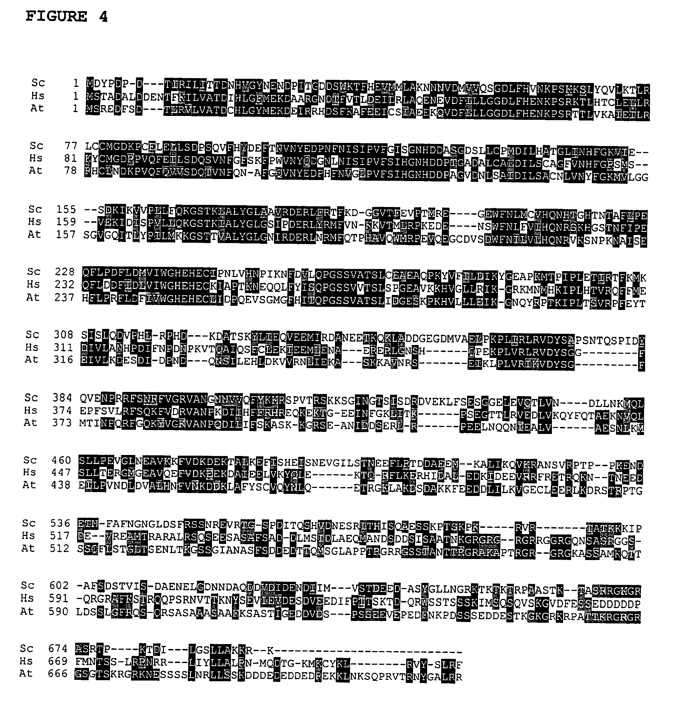

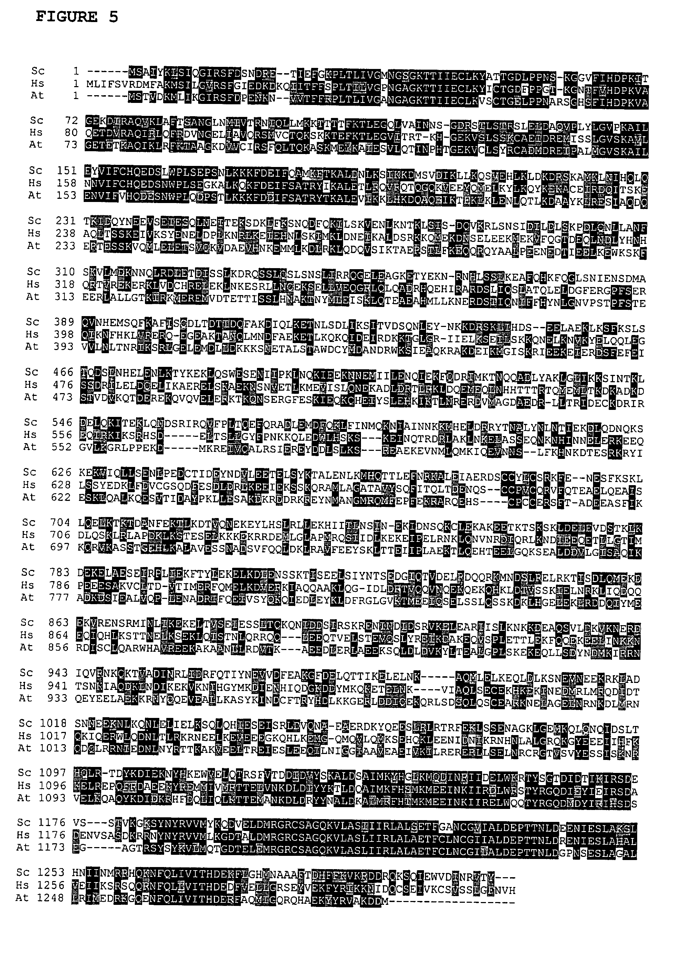

In a preferred embodiment the invention provides a method to direct nucleic acid integration to a pre-determined site or to a sub-telomeric and/or telomeric region by providing a mutant of a component involved in non-homologous recombination or by partially or more preferably completely inhibiting a component involved in non-homologous recombination wherein said component comprises *ku70*, *rad50*, *mre11*, *xrs2*, *lig4* or *sir4.* Components involved in non-homologous recombination are identified as outlined in the present description. The nomenclature for genes as used above is specific for yeast. Because the nomenclature of genes differs between organisms a functional equivalent or a functional homologue (see for example figure 2 to 5) and/or a functional fragment thereof, all defined herein as being capable of performing (in function, not in amount) at least one function of the yeast genes *ku70*, *rad50*, *mre11*, *xrs2*, *lig4* or *sir4* are also included in the present invention. A mutant of a component directly associating with a component involved in non-homologous recombination or (partial or complete) inhibition of a component directly associating with a component involved in non-homologous recombination is also part of this invention. Such a component directly associating with a component involved in non-homologous recombination is, for example, identified in a yeast two hybrid screening. An example of a component directly associating with a component involved in non-homologous recombination is *KU80,* which forms a complex with *KU70.* In a more preferred embodiment the invention provides a method to direct nucleic acid integration in yeast, fungus, plant or animal.

In another embodiment the invention provides a method to direct nucleic acid integration to a pre-determined site, whereby said nucleic acid has homology at or around the said pre-determined site, in an eukaryote with a preference for non-homologous recombination comprising steering an integration pathway towards homologous recombination by transiently (partially or more preferably completely) inhibiting integration via non-homologous recombination. In yet another embodiment the invention provides a method to direct integration of a nucleic acid of interest to a sub-telomeric and/or telomeric region in an eukaryote with a preference for non-homologous recombination by transiently (partially or more preferably completely) inhibiting integration via non-homologous recombination. In a more preferred embodiment such a method is used for yeast, plant or fungus and the transient (partial or more preferable complete) inhibition is provided by an *Agrobacterium* Vir-fusion protein capable of (partially or more preferably completely) inhibiting a component involved in non-homologous recombination or capable of (partially or more preferably completely) inhibiting a functional equivalent or homologue thereof or capable of (partially or more preferably completely) inhibiting a component directly associating with a component involved in non-homologous recombination. In a even more preferred embodiment such a *Agrobacterium* Vir fusion protein comprises VirF or VirE2. It was shown that the *Agrobacterium* VirF and VirE2 proteins are directly transferred from *Agrobacterium* to plant cells during plant transformation (Vergunst et al. 2000). To, for example, accomplish T-DNA integration by HR in plants, VirF fusion proteins containing for example a peptide inhibitor of IR in plant cells are introduced concomitantly with the targeting T-DNA. It has been reported that the C-terminal part (approximately 40 amino acids) of VirF or VirE2 is sufficient to accomplish transfer of T-DNA. A functional fragment and/or a functional equivalent of VirF or VirE is therefore also included in the present invention. In an even more preferred embodiment a component involved in non-homologous recombination comprises *ku70*, *rad50*, *mre11*, *xrs2*, *lig4* or *sir4* or functional equivalents or homologous thereof or associating components. The nomenclature for genes as used above is specific for yeast. Because the nomenclature of genes differs between organisms a functional equivalent or a functional homologue (see for example figure 2 to 5) and/or a functional fragment thereof, all defined herein as being capable of performing (in function, not in amount) at least one function of the yeast genes *ku70*, *rad50*, *mre11*, *xrs2*, *lig4* or *sir4* are also included in the present invention. By transiently (partially or more preferably completely) inhibiting a component involved in non-homologous recombination a nucleic acid is integrated at any position without permanently modifying a component involved in non-homologous recombination and preventing unwanted side effects caused by the permanent presence of such a modified component involved in non-homologous recombination.

Methods according to the present invention, as extensively discussed above, are used in a wide variety of applications. One embodiment of the present invention is the replacement of an active gene by an inactive gene according to a method of the invention. Complete inactivation, which can usually not be accomplished by existing methods such as antisense technology or RNAi technology, is useful for instance for the inactivation of genes controlling undesired side branches of metabolic pathways, for instance to increase the quality of bulk products such as starch, or to increase the production of specific secondary metabolites or to inhibit formation of unwanted metabolites. Also to inactivate genes controlling senescence in fruits and flowers or that determine flower pigments. Another embodiment of the present invention is the replacement of an inactive gene by an active gene. One example is the replacement of an defect p53 by an intact p53. Many tumors acquire a mutation in p53 during their development which renders it inactive and often correlates with a poor response to cancer therapy. By replacing the defect p53 by an intact p53, for example via gene therapy, conventional anti cancer therapy have better changes of succeeding. In yet another embodiment of the invention a therapeutic proteinaceous substance is integrated via a method of the invention. In this way a tumoricidal gene can be delivered to a pre-determined site present only in e.g. proliferating cells, or present only in tumor cells, e.g. to the site where a tumor antigen is expressed from. In yet another embodiment the invention provides a method to introduce a substance conferring resistance for an antibiotic substance to a cell according to a method of the invention. Also a method according to the invention is used to confer a desired property to an eukaryotic cell. In an preferred embodiment method a gene delivery vehicle is used to deliver a desired nucleic acid to a pre-determined site. Gene delivery vehicles are well known in the art and include adenoviral vehicles, retroviral vehicles, non-viral vehicles such as liposomes, etc.. In this way, a for example, tumoricidal gene can be delivered to a pre-determined site present only in e.g. proliferating cells, or present only in tumor cells, e.g. to the site where a tumor antigen is expressed from.

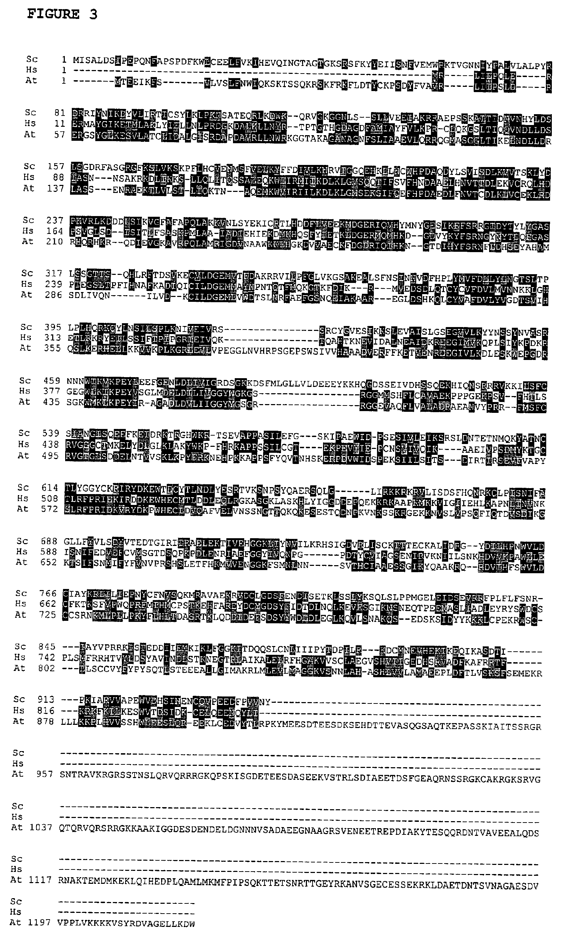

Furthermore a method according to the invention is used to improve gene targeting efficiency. Such a method is used to improve for example the gene targeting efficiency in plants. In plants transgenes integrate randomly into the genome by IR (Mayerhofer et al. 1991, Gheysen et al. 1991). The efficiency of integration by HR is very low, even when large stretches of homology between the transgene and the genomic target site are present (Offringa et al. 1990). Therefore, the efficiency of gene targeting using HR is very low in plants. The results that are disclosed herein show how to improve the gene targeting efficiency in plants. From the fact that T-DNA integration by IR is strongly reduced in *KU70*, *RAD50*, *MRE11*, *XRS2*, *LIG4* and *SIR4* deficient yeast strains and T-DNA integration by HR is not affected in such strains, we infer that also in plants, deficient for either of these genes, T-DNA integration by HR is more easily obtained. Recently, we have cloned a *KU70* homologue of *Arabidopsis thaliana* (see figure 2, Bundock 2000, unpublished data). *RAD50, MRE11* and *LIG4* homologues have already been found in *A.thaliana* (GenBank accession numbers AF168748, AJ243822 and AF233527, respectively, see also figure 3, 4 and 5 (Hartung and Puchta 1999). Currently, screenings are being performed to find plants carrying a T-DNA inserted in *AtMRE11*, *AtKU70* or *AtLIG4*. These knockout plants are used to test whether T-DNA integration by IR is reduced and integration by HR is unaffected, thereby facilitating the detection of T-DNA integration by HR.

The invention will be explained in more detail in the following description, which is not limiting the invention.

### EXPERIMENTAL PART

### Yeast strains.

The yeast strains that were used are listed in Table 1. Yeast mutants isogenic to the haploid YPH250 strain were constructed using the one-step disruption method (Rothstein 1991). A 1987 bp fragment from the *YKU70* locus was amplified by PCR using the primers hdf1p1 5'-GGGATTGCTTTAAGGTAG-3' and hdf1p2 5'-CAAATACCCTACCCTACC-3'. The PCR product was cloned into pT7Blue (Novagen) to obtain pT7Blue *YKU70*. A 1177 bp *EcoR*V/*Hin*dIII fragment from the *YKU70* ORF was replaced by a 2033 bp *Hin*dIII/*Smα*I *LEU2* containing fragment from pJJ283 (Jones and Prakash 1990), to form pT7Blue *YKU70::LEU2.* In order to obtain YKU70 disruptants Leu⁺ colonies were selected after transformation of YPH250 with a 2884 bp *Nde*I/*Smα*I fragment from pT7Blue *YKU70::LEU2.* The Expand™ High Fidelity System (Boehringer Mannheim) was used according to the supplied protocol to amplify a 3285 bp fragment from the *LIG4* locus with primers dnl4p1 5'-CGTAAGATTCGCCGAGTATAG-3' and dnl4p2 5'-CGTTTCAAATGGGACCACAGC-3'. The PCR product was cloned into pGEMT (Promega), resulting in pGEMT*LIG4*. A 1326 bp *Bam*HI/*Xho*I fragment from pJJ215 (Jones and Prakash 1990) containing the *HIS3* gene was inserted into the *Bam*HI and *Xho*I sites of pIC20R, resulting in pIC20R*HIS3*. A 782 bp *Eco*RI fragment from the *LIG4* ORF was replaced with a 1367 bp EcoRI *HIS3* containing fragment from pIC20R*HIS3* to construct pGEMT*LIG4::HIS3*. In order to obtain LIG4 disruptants His⁺ colonies were selected after transformation of YPH250 with a 3854 bp *Nco*I/*Not*I fragment from pGEMT*LIG4::HIS3*. In order to obtain RAD50 disruptants YPH250 was transformed with a *Eco*RI/*Bgl*II fragment from pNKY83 and Ura⁺ colonies were selected (Alani et al. 1989). A *rad50::hisG* strain was obtained by selecting Ura- colonies on selective medium containing 5-FOA. Similarly RAD51 disruptants were obtained were obtained after transformation of YPH250 with a *RAD51::LEU2 Xba*I/*Pst*I fragment from pDG152 and selection of Leu⁺ colonies (Schiestl et al. 1994). The *TRP1* marker in pSM21 (Schild et al. 1983) was replaced with a *Bgl*II/*Xba*I *LEU2* containing fragment from pJJ283 (Jones and Prakash, 1990). This resulted in pSM21*LEU2*. Leu⁺ RAD52 disruptant colonies were selected after transformation of YPH250 with the *RAD52::LEU2 BamHI* fragment from pSM21*LEU2*. Disruption constructs were transformed to YPH250 by the lithium acetate transformation method as described (Gietz et al. 1992). Disruption of *YKU70*, *LIG4*, *RAD50*, *RAD51* and *RAD52* was confirmed by PCR and Southern blot analysis.

**Table 1:**

| Yeast strains | | |
|---|---|---|
| Strain | Genotype | Reference |
| YPH250 | *MATα*, *ura3-52*, *lys2-801*, *ade2-101*, *trp1-Δ1*, *his3-Δ200*, *leu2-Δ1* | Sikorski and Hieter 1989 |
| YPH250*rad51* | *MATα*, *ura3-52*, *lys2-801*, *ade2-101*, *trp1-Δ1*, *his3-Δ200*, *leu2-Δ1*, *rad51::LEU2* | This study |
| YPH250*rad52* | *MATα*, *ura3-52*, *lys2-801*, *ade2-101*, *trp1-Δ1*, *his3-Δ200*, *leu2-Δ1*, *rad52::LEU2* | This study |
| YPH250*yku70* | *MATα*, *ura3-52*, *lys2-801*, *ade2-101*, *trp1-Δ1*, *his3-Δ200*, *leu2-Δ1*, *yku70::LEU2* | This study |
| YPH250*rad50* | *MATα*, *ura3-52*, *lys2-801*, *ade2-101*, *trp1-Δ1*, *his3-Δ200*, *leu2-Δ1*, *rad50::hisG* | This study |
| YPH250*lig4* | *MATα*, *ura3-52*, *lys2-801*, *ade2-101*, *trp1-Δ1*, *his3-Δ200*, *leu2-Δ1*, *lig4::HIS3* | This study |
| JKM115 | *Δho*, *Δhml::ADE1*, *MATα*, *Δhmr::ADE1*, *ade1*, *leu2-3,112*, *Iys5, trp1::hisG, ura3-52* | Moore and Haber 1996 |
| JKM129 | Δ*ho*, *Δhml::ADE1*, *MATα*, *Δhmr::ADE1*, *ade1*, *leu2-3,112*, *lys5*, *trp1::hisG, ura3-52*, *xrs2::LEU2* | Moore and Haber 1996 |
| JKM138 | *Δho*, *Δhml::ADE1, MATα*, *Δhmr::ADE1, ade1*, *leu2-3,112*, *lys5*, *trp1::hisG, ura3-52*, *mre11::hisG* | Moore and Haber 1996 |
| YSL204 | *Δho*, *HMLα*, *MATα*, *HMRα*, *ade1-100, leu2-3,112, lys5, trp1::hisG*, *ura3-52*, *hisG'-URA3-hisG'*, *sir4::HIS3* | Lee et al. 1999 |

### Construction of binary vectors.

To construct pSDM8000 a 1513 bp *Pvu*II/*Eco*RV fragment carrying the *KanMX* marker was obtained from pFA6a (Wach et al. 1994) and was ligated into the unique *HpaI* site of pSDM14 (Offringa 1992). pSDM8001 was made in three cloning steps. A 1476 bp *Bam*HI/*Eco*RI fragment carrying the *KanMX* marker was obtained from pFA6a and ligated into *Bam*HI and *Eco*RI digested pIC20H to form pIC20H*kan*MX. The KanMX marker was inserted between the *PDA1* flanks by replacement of a 2610 bp *Bgl*II fragment from pUC4E1α10 (Steensma et al. 1990) with a 1465 *Bgl*II fragment from pIC20H*kan*MX. A 3721 bp *Xho*I/*Kpn*I fragment from this construct was inserted into the *Xho*I and *Kpn*I sites of pSDM14. The binary vectors pSDM8000 and pSDM8001 were introduced into *Agrobacterium tumefaciens* LBA1119 by electroporation (den Dulk-Ras and Hooykaas 1995).

### Cocultivations / T-DNA transfer experiments.

Cocultivations were performed as described earlier with slight modifications (Bundock et al. 1995). *Agrobacterium* was grown overnight in LC medium. The mix *of Agrobacterium* and *S*. *cerevisiae* cells was incubated for 9 days at 20°C. G418 resistant *S.cerevisiae* strains were selected at 30°C on YPAD medium containing geneticin (200 µg/ml) (Life Technologies/Gibco BRL).

### Vectorette PCR.

Chromosomal DNA was isolated using Qiagen's Genomic Tips G/20 per manufacters protocol. 1-2 µg of Genomic DNA was digested with *Eco*RI, *Clα*I, *Pst*I or *Hin*dIII and run on a 1% TBE-gel. Non-radioactive Southern blotting was performed. The membrane was hybridized with a digoxigenine-labeled kanMX probe to determine the size of T-DNA/genomic DNA fragments (*Eco*RI and *Cla*I for RB containing fragments and *Pst*I and *Hin*dIII for LB containing fragments). The kanMX probe, a 792 bp internal fragment of the *KanMX* marker, was made by PCR using primers kanmxp1 5'-AGACTCACGTTTCGAGGCC-3' and kanmxp2 5'-TCACCGAGGCAGTTCCATAG-3' and a Non-Radioactive DNA Labeling and Detection kit (Boehringer Mannheim). The enzyme showing the smallest band on blot was used for Vectorette PCR, in order to amplify the smallest junction sequence of T-DNA and genomic DNA. Vectorette PCR was performed as described
(http://genomewww.stanford.edu/group/botlab/protocols/vectorette.html). The Expand™ High Fidelity System (Boehringer Mannheim) was used to amplify fragments larger than 2.5 kb, whereas sTaq DNA polymerase (SphaeroQ) was used for amplification of fragments smaller than 2.5 kb. Primer kanmxp3 5'-TCGCAGGTCTGCAGCGAGGAGC-3' and kanmxp4 5'-TCGCCTCGACATCATCTGCCCAG-3' were used to amplify RB/genomic DNA and LB/genomic DNA junction sequences, respectively.

### T7 DNA Polymerase sequencing.

Vectorette PCR products were cloned in pGEMTEasy (Promega) and sequenced using the T7 polymerase sequencing kit (Pharmacia) according to manufacturers protocol. In order to obtain sequences flanking the RB and LB, primers kanmxp5 5'-TCACATCATGCCCCTGAGCTGC-3' and kanmxp4 were used, respectively.

### RESULTS

### 1. Binary vectors for T-DNA transfer to yeast.

It was previously demonstrated that *Agrobacterium tumefaciens* is able to transfer its T-DNA not only to plants but also to another eukaryote, namely the yeast *Saccharomyces cerevisiae* (Bundock et al. 1995). T-DNA carrying homology with the yeast genome was shown to become integrated by homologous recombination. T-DNA lacking any homology with the yeast genome was integrated randomly into the genome by IR like in plants (Bundock et al. 1995, Bundock and Hooykaas 1996). The T-DNA used in these experiments carried the *S.cerevisiae URA3* gene for selection of Ura⁺ colonies after T-DNA transfer to the haploid yeast strain RSY12(*URA3*Δ). However, in this system only yeast strains could be used in which the URA3 gene had been deleted to avoid homology between the incoming T-DNA and the *S.cerevisiae* genome.

We wanted to setup a system in which T-DNA transfer to any yeast strain could be studied. Therefore, two new binary vectors were constructed using the dominant marker *kanMX* (Wach et al. 1994), which confers resistance against geneticin (G418). The T-DNA of pSDM8000 carries only the *KanMX* marker. Since this *KanMX* marker consists of heterologous DNA, lacking any homology with the *S.cerevisiae* genome, we would expect this T-DNA to integrate by IR at a random position in the yeast genome. To be able to compare this with T-DNA integration by homologous recombination pSDM8001 was constructed. The T-DNA of pSDM8001 carries the *KanMX* marker flanked by sequences from the *S.cerevisiae PDA1* locus. The *PDA1* sequences have been shown to mediate the integration of T-DNA by HR at the *PDA1* locus on chromosome V (Bundock et al. 1995).

Cocultivations between *Agrobacterium* strains carrying pSDM8000 and pSDM8001, respectively, and the haploid yeast strains YPH250 and JKM115, respectively, were carried out as described in the experimental part. G418 resistant colonies were obtained at low frequencies for YPH250 (1.6 x 10⁻⁷) and JKM115 (1.2 x 10⁻⁵) after T-DNA transfer from pSDM8000 (Table 2). T-DNA transfer from pSDM8001 generated G418 resistant colonies at higher frequencies (2.4 x 10⁻⁵ for YPH250 and 1.8 x 10⁻⁴ JKM115, Table 2). The ratio of homologous recombination versus illegitimate recombination is determined by comparing the frequencies of G418 resistant colonies obtained from cocultivations using either pSDM8001 or pSDM8000. This showed that a T-DNA from pSDM8001 was 150-fold more likely to integrate than a T-DNA from pSDM8000 in YPH250 (Table 2). A similar difference was previously seen using T-DNAs with the *URA3* marker (Bundock and Hooykaas 1996). In contrast, T-DNA from pSDM8001 was only 16-fold more likely to integrate than a T-DNA from pSDM8000 in JKM115. There was no significant difference in the frequency of T-DNA transfer to these two yeast strains as was determined by T-DNA transfer experiments in which a T-DNA, that carried the *KanMX* marker and the yeast 2 micron replicon, was employed. Therefore, the differences in the frequencies of T-DNA integration by HR and IR between the yeast strains YPH250 and JKM115, respectively, is most likely contributed to differences in the capacities of their HR and IR recombination machineries.

We confirmed by PCR that the T-DNA from pSDM8001 became integrated at the *PDA1* locus by homologous recombination (data not shown). In order to find out whether the T-DNA from pSDM8000 had integrated randomly by IR yeast target sites for integration were determined from 8 G418 resistant YPH250 colonies by Vectorette PCR (for detailed description see materials and methods). Chromosomal DNA was isolated and digested with a restriction enzyme that cuts within the T-DNA. A Vectorette was ligated to the digested DNA and a PCR was performed using a T-DNA specific and a Vectorette specific primer. The PCR product obtained was cloned into pGEMTEasy and sequenced using a T-DNA specific primer. The position of the T-DNA insertion was determined by basic BLAST search of the yeast genome (http://www-genome.stanford.edu/SGD). We were thus able to map the position of the T-DNA insertions of all 8 G418 resistant colonies analyzed. They were present at different positions spread out over the genome. Comparison of the T-DNA sequence and yeast target site sequences did not reveal any obvious homology. These data show that the T-DNA from pSDM8000 had integrated via an IR mechanism as expected.

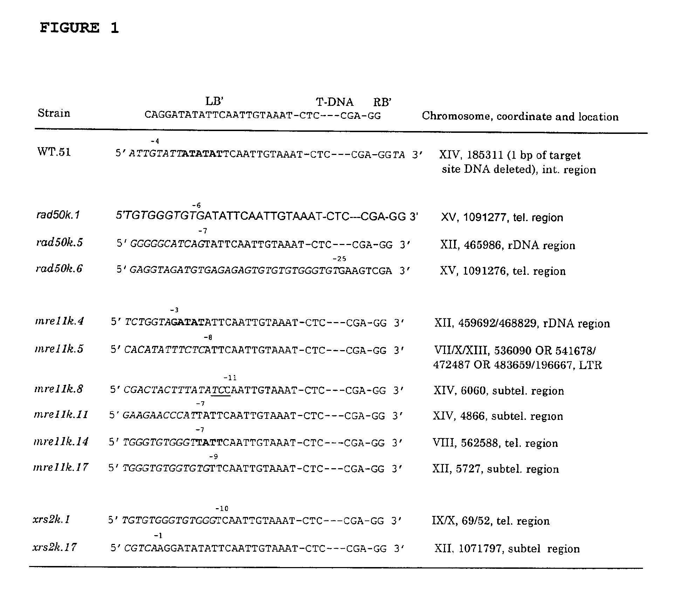

The following characteristics have previously been observed for T-DNAs integrated by IR: a) the 3' end of the T-DNA is usually less conserved compared to the 5' end, b) microhomology is sometimes present between the T-DNA ends and the target site, c) integration is often accompanied by small deletions of the target site DNA (Matsumoto et al. 1990, Gheysen et al. 1991, Mayerhofer et al. 1991, Bundock and Hooykaas 1996). Similar characteristics were seen in the currently analyzed 8 T-DNA insertions. In 3 strains we observed microhomology of 2 - 6 bp between the LB and yeast target site (figure 1, WT.51 was taken as an example). In 5 strains deletions of 1 - 5 bp of yeast target site DNA was found and we observed deletions, varying from 1 - 112 bp, of the 3' end of the T-DNA in 7 out of 8 analyzed strains. In only 1 strain the 3' end appeared to be intact. The 5' end of the T-DNA was conserved in almost all strains. In only 2 strains we observed small deletions of 1 and 2 bp at the 5' end of the T-DNA.

Thus, we can conclude that the T-DNA from pSDM8000 had integrated via the same IR mechanism described before.

**Table 2:**

| frequencies of T-DNA integration by IR relative to integration by HR in recombination defective yeast strains | | | | | |
|---|---|---|---|---|---|
| Strain | Genotype | Freq. of IR^{a} | Freq. of HR | Absolute IR/HR ratio^{b} | Standardized IR/HR ratio^{c} |
| YPH250 | WT | 1.6 x 10⁻⁷ | 2.4 x 10⁻⁵ | 0.007 | 1 |
| YPH250 *rad51* | *rad51Δ* | 1.4 x 10⁻⁷ | 1.5 x 10⁻⁶ | 0.09 | 14 |
| YPH250 *rad52* | *rad52Δ* | 3.8 x 10⁻⁷ | 2.5 x 10⁻⁶ | 0.15 | 23 |
| YPH250 *yku70* | *yku70Δ* | <3.2 x 10⁻⁹ | 3.3 x 10⁻⁵ | <0.0001 | <0.01 |
| YPH250 *rad50* | *rad50Δ* | 8.0 x 10⁻⁹ | 3.5 x 10⁻⁵ | 0.0002 | 0.03 |
| YPH250 *lig4* | *lig4*Δ | 3.7 x 10⁻⁹ | 2.3 x 10⁻⁵ | 0.0002 | 0.02 |
| | | | | | |
| JKM115 | WT | 1.2 x 10⁻⁵ | 1.8 x 10⁻⁴ | 0.07 | 1 |
| JKM129 | *xrs2*Δ | 2.7 x 10⁻⁷ | 5.1 x 10⁻⁵ | 0.005 | 0.08 |
| JKM138 | *mre11Δ* | 2.9 x 10⁻⁷ | 7.5 x 10⁻⁵ | 0.004 | 0.06 |
| YSL204 | *sir4Δ* | 1.5 x 10⁻⁷ | 1.8 x 10⁻⁵ | 0.008 | 0.13 |

| | | | | | |
|---|---|---|---|---|---|
| ^{a} Averages of 2 or more independant experiments are shown. Frequencies are depicted as the number of G418 resistant colonies devided by the output number of yeast cells (cells/ml). | | | | | |
| ^{b} The frequency of T-DNA integration by IR (pSDM8000) devided by the frequency of T-DNA integration by HR (pSDM8001). | | | | | |
| ^{c} The ratio of IR/HR in the mutant strain devided by the ratio of IR/HR in the wildtype strain. | | | | | |

### 2. Host-specific factors involved in random T-DNA integration.

The observation that the T-DNA from pSDM8000 integrates by IR into the yeast genome allowed us to use this system to study the effect of host factors on the process of integration. Many proteins involved in various forms of DNA recombination have been identified in yeast. In order to determine the roles of a representative set of these enzymes in T-DNA integration, we compared T-DNA transfer and integration in wildtype yeasts with that of strains carrying disruptions of the genes encoding several recombination proteins. The *RAD51*, *RAD52*, *KU70, RAD50* and *LIG4* genes were deleted from YPH250 using the one step disruption method. Yeast strains carrying deletions in *MRE11*, *XRS2* and *SIR4* in the JKM115 background were kindly provided by Dr. J. Haber. The results of cocultivations with these yeast strains are shown in Table 2.

In *rad51* and *rad52* mutants, which are impaired in homologous recombination, the frequency of T-DNA integration by HR was 16- and 9-fold lower, respectively, than observed for the wildtype (Table 2). This showed that *RAD51* and *RAD52* play a role in T-DNA integration by homologous recombination. In the IR defective *ku70*, *rad50*, *lig4*, *mre11*, *xrs2* and *sir4* mutants the frequency of T-DNA integration by HR did not differ significantly from that observed for wildtype (Table 2). This showed that these genes do not play a role in T-DNA integration by homologous recombination.

The frequency of T-DNA integration by IR in a *rad51* mutant did not differ significantly from that observed for wildtype, whereas in a *rad52* mutant the frequency was about 2-fold higher (Table 2). This showed that *RAD51* and *RAD52* are not involved in T-DNA integration by IR. The product of the *RAD52* gene may compete with IR-enzymes for the T-DNA and thereby inhibits integration by IR to some extent. Strikingly, in *rad50*, *mre11*, *xrs2, lig4* and *sir4* mutants the frequency of T-DNA integration by IR was reduced dramatically: 20- to more than 40-fold (Table 2). T-DNA integration by IR seemed to be completely abolished in the *ku70* mutant. We did not obtain any G418 resistant colonies from several cocultivation experiments. This strongly suggests that KU70 plays an important role in random T-DNA integration in yeast.

Since T-DNA integration by HR is normal in these mutants, these results clearly show that the yeast genes *KU70*, *RAD50*, *MRE11*, *XRS2*, *LIG4* and *SIR4* are involved in T-DNA integration by illegitimate recombination.

### 3. Chromosomal distribution of integrated T-DNA copies in IR defective S.cerevisiae.

From several cocultivation experiments with the *rad50*, *mre11*, *xrs2, lig4* and *sir4* mutants we obtained a small number of G418 resistant colonies. The T-DNA structure was determined for a number of these lines. To this end chromosomal DNA was isolated from these G418 resistant colonies and subjected to vectorette PCR to amplify junction sequences of genomic DNA and T-DNA. PCR products were cloned and sequenced. The yeast sequences linked to the T-DNA were used in a BLAST search at http://www-genome.stanford.edu/SGD to determine the T-DNA integration sites.

Strikingly, analysis of LB/genomic DNA junctions revealed that in 2 out of 3 *rad50*, 4 out of 6 *mre11* and 2 *xrs2* strains analyzed, T-DNAs had integrated in telomeres or subtelomeric regions (*rad50k.1*, *rad50k.6*, *mre11k.8*, *mre11k.11*, *mre11k.14*, *mre11k.17*, *xrs2k.1* and *xrs2k.17*; Table 3 and figure 1). *S*. *cerevisiae* telomeres generally consist of one or more copies of the Y' element followed by telomerase-generated C(1-3)A/TG(1-3) repeats (Zakian 1996). In 2 *rad50* strains, *2 mre11* strains and 1 *xrs2* strain the LB was found to be fused to this typical telomerase-generated C(1-3)A/TG(1-3) repeat (*rad50k.1, rad50k.6*, *mre11k.14*, *mre11k.17* and *xrs2k.1*; figure 1). Besides, we also found one T-DNA insertion in a Ty LTR element in the *mre11* mutant and 2 insertions in the rDNA region, present in chromosome XII, in the *mre11* and *rad50* mutants (*mre11k.5*, *mre11k.4* and *rad50k.5*, respectively; Table 3 and figure 1).

The 3' end of the T-DNA was truncated in all strains. Deletions of 3 - 11 bp of the 3'end of the T-DNA were observed (figure 1). Microhomology between the 3' end of the T-DNA and yeast target site was only found in 2 lines (5 bp in *mre11k.4* and 4 bp in *mre11k.14;* figure 1). For the T-DNA copies present at the yeast telomeres, the RB/genomic DNA junction sequences could not be obtained from these strains using vectorette PCR. This was only possible for the *rad50* and *mre11* strains carrying the T-DNA in the rDNA region on chromosome XII. In both strains the RB was intact and no homology between the 5' end of the T-DNA and the yeast target site was found (data not shown in figure 1).

Previously, target sites for T-DNA integration in the genome of *S.cerevisiae* strain RSY12 were determined (Bundock and Hooykaas 1996, Bundock 1999). In 4 out of 44 strains analyzed, T-DNA copies were integrated. in rDNA, Ty LTR elements (in 2 strains) and a subtelomeric located Y' element, respectively. In addition, we determined the position of T-DNA integration in ten *S.cerevisiae* YPH250 strains. We did not find any T-DNA insertions in rDNA, LTR elements or subtelomeric/telomeric regions amongst these ten. Pooling all insertions analyzed in wildtype (54), in 2 out of 54 strains analyzed (4%) insertions were found in a Ty LTR element and in two other strains in the rDNA repeat (2%) and a subtelomeric region (2%), respectively. In contrast, we report here that T-DNA in yeast strains mutated in *RAD50*, *MRE11* or *XRS2* T-DNA integrates preferentially in (sub)telomeric regions (8 out of 11 lines: ~73%) of *rad50*, *mre11* and *xrs2* mutants (table 3). From the remaining strains two T-DNAs were present in rDNA and one in a Ty LTR element, respectively. Apparently, the rDNA repeat is also a preferred integration site in these mutants (~18% vs. ~2% in the wildtype).

Telomeres consist of a large array of telomerase-generated C(1-3)A/TG(1-3) repeats (~350 bp). In the subtelomeric regions two common classes of Y' elements, 6.7 and 5.2 kb, can be found (in most strains chromosome I does not contain Y') (Zakian and Blanton 1988), making the average size of these regions ~6,0 kb. Thus, the yeast genome contains (16 x 2 x 0.35) + (15 x 2 x 6,0) = 191 kb of subtelomeric/telomeric sequences. The yeast genome is 12,052 kb in size, which means that only 1.6% of the genome consists of subtelomeric/telomeric sequences. In accordance with this, we observed in only 2% of the wildtype strains T-DNA copies inserted in a subtelomeric region, which we would expect on the basis of random T-DNA integration. In contrast, in the *rad50*, *mre11* and *xrs2* mutants 73% of the T-DNA insertions were found in the (sub)telomeric region.

Analysis of 7 lines revealed that in the *sir4* mutant T-DNA was integrated randomly into the yeast genome. So, although *SIR4* has an effect on the efficiency of T-DNA integration by IR, the pattern of T-DNA distribution in the transformants seems similar as in the wildtype strain. In the *sir4* mutant T-DNA integration by IR was characterized by truncation of the 3' end of the T-DNA, deletions at the target site and microhomology between the LB and the target site (data not shown), like this was observed for T-DNA integration by IR in the wildtype.

These results clearly show that in the *rad50*, *mre11* and *xrs2* mutants the T-DNA, if integrated at all, becomes preferentially inserted in telomeres or subtelomeric regions and that the genomic distribution of integrated T-DNAs is altered when compared to wildtype. However, disruption of *SIR4* did affect the efficiency of T-DNA integration by IR, but not the characteristics of this process.

**Table 3:**

| genomic distribution of T-DNA integrated by IR in *rad50*, *mre11* and *xrs2* mutants in comparison with the wildtype after T-DNA transfer from pSDM8000 | | | | |
|---|---|---|---|---|
| *Yeast strain* | *(Sub)Telomeric region* | *LTR* | *rDNA* | Elsewhere |
| *rad50* mutant | 2 | 0 | 1 | 0 |
| *mre11* mutant | 4 | 1 | 1 | 0 |
| *xrs2* mutant | 2 | 0 | 0 | 0 |
| wildtype | 1 | 2 | 1 | 50 |

### DESCRIPTION OF FIGURES

Figure 1: Junction sequences of T-DNA and *S.cerevisae* genomic DNA. *S.cerevisiae* YPH250 (WT), *rad50*, *mre11* and *xrs2* strains were cocultivated with LBA1119(pSDM8000). G418 resistant colonies were obtained. Chromosomal DNA was isolated and subjected to Vectorette PCR to determine the sequence of genomic DNA flanking the T-DNA. Position of T-DNA integration was determined by basic BLAST search of the yeast genome at http:/www.genome-stanford.edu/SGD. The Watson strand of genomic DNA that is fused to the LB or RB is shown in italics. Bold sequences represent sequence homology between the LB and target site. The filler DNA sequence is underlined and depicted in italics. The numbers above the LB sequences represents the number of bp deleted from the LB. Tel. = telomeric, Subtel. = subtelomeric and Int. = intergenic.

Figure 2: Alignment of KU70 homologues. Sc = *Saccharomyces cerevisiae,* Hs = *Homo sapiens* and At = *Arabidopsis thaliana.* Perfect identity is depicted as black boxes, homology is depicted as grey boxes and dashes were used to optimise alignment.

Figure 3: Alignment of LIG4 homologues. Sc = *Saccharomyces cerevisiae*, Hs *= Homo sapiens* and At = *Arabidopsis thaliana*. Perfect identity is depicted as black boxes, homology is depicted as grey boxes and dashes were used to optimise alignment.

Figure 4: Alignment of MRE11 homologues. Sc *= Saccharomyces cerevisiae*, Hs *= Homo sapiens* and At *= Arabidopsis thaliana*. Perfect identity is depicted as black boxes, homology is depicted as grey boxes and dashes were used to optimise alignment.

Figure 5: Alignment of RAD50 homologues. Sc = *Saccharomyces cerevisiae*, Hs *= Homo sapiens* and At = *Arabidopsis thaliana*. Perfect identity is depicted as black boxes, homology is depicted as grey boxes and dashes were used to optimise alignment.

### REFERENCE LIST

Alani, E., Subbiah, S., and Kleckner, N. 1989. The yeast *RAD50* gene encodes a predicted 153-kD protein containing a purine nucleotide-binding domain and two large heptad-repeat regions. Genetics 122:47-57.

Bundock, P. 1999. *Agrobacterium tumefaciens*-mediated transformation of yeast and fungi. Thesis Leiden University.

Bundock, P., den Dulk-Ras, A., Beijersbergen, A., and Hooykaas, P. J. J. 1995. Trans-kingdom T-DNA transfer from *Agrobacterium tumefaciens* to *Saccharomyces cerevisiae.* EMBO J. 14:3206-3214.

Bundock, P. and Hooykaas, P. J. J. 1996. Integration of *Agrobacterium tumefaciens* T-DNA in the *Saccharomyces cerevisiae* genome by illegitimate recombination. Proc. Natl. Acad. Sci U.S.A 93:15272-15275.

deGroot, M. J. A., Bundock, P., Hooykaas, P. J. J., and Beijersbergen, A. G. M. 1998. *Agrobacterium tumefaciens-mediated* transformation of filamentous fungi. Nat. Biotech. 16:839-842.

den Dulk-Ras, A. and Hooykaas, P. J. J. 1995. Electroporation of *Agrobacterium tumefaciens*. Methods Mol. Biol. 55:63-72.

Gheysen, G., Villarroel, R., and Van Montagu, M. 1991. Illegitimate recombination in plants: a model for T-DNA integration. Genes Dev. 5:287-297.

Gietz, D., St.Jean, A., Woods, R. A., and Schiestl, R. H. 1992. Improved method for high efficiency transformation of intact yeast cells. Nucleic Acids Res. 20:1425.

Hansen, G. and Chilton, M. D. 1999. Lessons in gene transfer to plants by a gifted microbe. Curr. Top. Microbiol. Immunol. 240:21-57.

Hartung, F. and Puchta, H. 1999. Isolation of the complete cDNA of the *MRE11* homologue of *Arabidopsis* (Accession No. AJ243822) indicates conservation of DNA recombination mechanisms between plants and other eukaryotes. Plant Phys. 121:312-312.

Hooykaas, P. J. J. and Beijersbergen, A. G. M. 1994. The virulence system of *Agrobacterium tumefaciens.* Annu. Rev. of Phytopathol. 32:157-179.

Jones, J. S. and Prakash, L. 1990. Yeast *Saccharomyces cerevisiae* selectable markers in pUC18 polylinkers. Yeast 6:363-366.

Lee, S. E., Paques, F., Sylvan, J., and Haber, J. E. 1999. Role of yeast *SIR* genes and mating type in directing DNA double-strand breaks to homologous and non-homologous repair paths. Curr. Biol. 9:767-770.

Matsumoto, S., Ito, Y., Hosoi, T., Takahashi, Y., and Machida, Y. 1990. Integration of Agrobacterium T-DNA into a tobacco chromosome: possible involvement of DNA homology between T-DNA and plant DNA. Mol. Gen. Genet. 224:309-316.

Mayerhofer, R., Koncz-Kalman, Z., Nawrath, C., Bakkeren, G., Crameri, A., Angelis, K., Redei, G. P., Schell, J., Hohn, B., and Koncz, C. 1991. T-DNA integration: a mode of illegitimate recombination in plants. EMBO J. 10:687-704.

Moore, J. K. and Haber, J. E. 1996. Cell cycle and genetic requirements of two pathways of nonhomologous end-joining repair of double-strand breaks in Saccharomyces cerevisiae. Mol. Cell. Biol. 16:2164-2173.

Offringa, R. 1992. Gene targeting in plants using the *Agrobacterium* vector system. Thesis Leiden University.

Offringa, R., de Groot, M. J., Haagsman, H. J., Does, M. P., van den Elzen, P. J., and Hooykaas, P. J. J. 1990. Extrachromosomal homologous recombination and gene targeting in plant cells after *Agrobacterium* mediated transformation. EMBO J. 9:3077-3084.

Rothstein, R. 1991. Targeting, disruption, replacement, and allele rescue: integrative DNA transformation in yeast. Methods Enzymol. 194:281-301.

Schiestl, R. H., Zhu, J., and Petes, T. D. 1994. Effect of mutations in genes affecting homologous recombination on restriction enzyme-mediated and illegitimate recombination in *Saccharomyces cerevisiae.* Mol. Cell. Biol. 14:4493-4500.

Schild, D., I. L. Calderon, C. R. Contopoulou, and R. K. Mortimer. 1983. Cloning of yeast recombination repair genes and evidence that several are nonessential genes, p. 417-427. *In*: E. C. Friedberg and B. A. Bridges (eds.), Cellular responses to DNA damage. Alan R. Liss Inc., New York.

Sikorski, R. S. and Hieter, P. 1989. A system of shuttle vectors and yeast host strains designed for efficient manipulation of DNA in *Saccharomyces cerevisiae.* Genetics 122:19-27.

Steensma, H. Y., Holterman, L., Dekker, I., van Sluis, C. A., and Wenzel, T. J. 1990. Molecular cloning of the gene for the El alpha subunit of the pyruvate dehydrogenase complex from *Saccharomyces cerevisiae.* Eur. J. Biochem. 191:769-774.

Vergunst, A. C., Schrammeijer, B., den Dulk-Ras, A., de Vlaam, C. M. T., Regensburg-Tuïnk, T. J. G., and Hooykaas, P. J. J. 2000, VirB/D4 dependent protein translocation from *Agrobacterium* into plant cells. Science: in press.

Wach, A., Brachat, A., Pohlmann, R., and Philippsen, P. 1994. New heterologous modules for classical or PCR-based gene disruptions in *Saccharomyces cerevisiae.* Yeast 10:1793-1808.

Zakian, V. A. 1996. Structure, function, and replication of *Saccharomyces cerevisiae* telomeres. Annu. Rev. Genet. 30:141-172.

Zakian, V. A. and Blanton, H. M. 1988. Distribution of telomere-associated sequences on natural chromosomes in *Saccharomyces cerevisiae.* Mol. Cell. Biol. 8:2257-2260.

Zrenner, R., Willmitzer, L., and Sonnewald, U. 1993. Analysis of the expression of potato uridinediphosphate-glucose pyrophosphorylase and its inhibition by antisense RNA. Planta, 190:247-252

## Claims

1. A method to direct integration of a nucleic acid of interest to a pre-determined site, whereby said nucleic acid has homology at or around the said pre-determined site, in an eukaryote with a preference for non-homologous recombination, comprising steering an integration pathway towards homologous recombination.

2. A method to direct nucleic acid integration according to claim 1, comprising providing a mutant of a component involved in non-homologous recombination.

3. A method to direct nucleic acid integration according to claim 1 or 2, comprising inhibiting a component involved in non-homologous recombination.

4. A method according to claim 2 or 3 wherein said component involved in non-homologous recombination comprises *ku70*, *rad50*, *mre11*, *xrs2*, *lig4* or *sir4*.

5. A method to direct integration of a nucleic acid of interest to a subtelomeric and/or telomeric region in an eukaryote with a preference for non-homologous recombination by providing a mutant of a component involved in non-homologous recombination.

6. A method to direct integration of a nucleic acid of interest to a subtelomeric and/or telomeric region in an eukaryote with a preference for non-homologous recombination, comprising inhibiting a component involved in non-homologous recombination

7. A method to direct integration according to claim 5 or 6 wherein said component involved in non-homologous recombination comprises *rad50*, *mre11* or *xrs2*.

8. A method according to anyone of claims 1 to 7 wherein said eukaryote comprises yeast.

9. A method according to anyone of claims 1-8 comprising transiently inhibiting integration via non-homologous recombination.

10. A method according claim 9 wherein said transiently inhibiting is provided by an *Agrobacterium* Vir-fusion protein capable of inhibiting a component involved in non-homologous recombination.

11. A method to direct nucleic acid integration according to claim 10 wherein said *Agrobacterium* Vir fusion protein comprises VirF or VirE2.

12. A method according to claim 10 or 11 wherein said component involved in non-homologous recombination comprises *ku70*, *rad50*, *mre11*, *xrs2*, *lig4* or *sir4.*

13. A method according to anyone of the aforegoing claims wherein said nucleic acid of interest comprises an inactive gene to replace an active gene.

14. A method according to anyone of claims 1-12, wherein said nucleic acid of interest comprises an active gene to replace an inactive gene.

15. A method according to anyone of claims 1-12, wherein said nucleic acid of interest encodes a therapeutic proteinaceous substance.

16. A method according to anyone of claims 1-12, wherein said nucleic acid of interest encodes a substance conferring resistance for an antibiotic substance to a cell.

17. A method according to anyone of claims 1-12, wherein said nucleic acid of interest confers a desired property to said eukaryotic cell.

18. A method according to anyone of the aforegoing claims wherein said nucleic acid of interest is part of a gene delivery vehicle.

19. Use of a method according to anyone of claims 1 to 18 for improvement of gene targeting efficiency.
